(19) Europäisches Patentamt
European Patent Office
Office européen des brevets

(11) EP 4 438 613 A1

(12) EUROPEAN PATENT APPLICATION
published in accordance with Art. 153(4) EPC

(43) Date of publication:
**02.10.2024 Bulletin 2024/40**

(21) Application number: **22897943.1**

(22) Date of filing: **25.11.2022**

(51) International Patent Classification (IPC):
***C07F 9/6558*** *(2006.01)* ***A61K 31/506*** *(2006.01)*
***A61P 35/00*** *(2006.01)*

(52) Cooperative Patent Classification (CPC):
**A61K 31/506; A61K 31/675; A61P 35/00; C07C 303/32; C07C 309/30; C07F 9/6558**

(86) International application number:
**PCT/CN2022/134410**

(87) International publication number:
**WO 2023/093861 (01.06.2023 Gazette 2023/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.11.2021 CN 202111425629**

(71) Applicant: **Nanjing Chia Tai Tianqing Pharmaceutical Co., Ltd.**
**Jiangsu 210046 (CN)**

(72) Inventors:
- **MA, Changyou**
  **Nanjing, Jiangsu 210046 (CN)**
- **ZHANG, Linlin**
  **Nanjing, Jiangsu 210046 (CN)**
- **FENG, Haiwei**
  **Nanjing, Jiangsu 210046 (CN)**
- **ZHAO, Tingli**
  **Nanjing, Jiangsu 210046 (CN)**
- **WU, Jian**
  **Nanjing, Jiangsu 210046 (CN)**
- **XU, Dan**
  **Nanjing, Jiangsu 210046 (CN)**
- **ZHU, Chunxia**
  **Nanjing, Jiangsu 210046 (CN)**
- **TIAN, Zhoushan**
  **Nanjing, Jiangsu 210046 (CN)**

(74) Representative: **Elzaburu S.L.P.**
**Paseo de la Castellana 259C**
**Torre de Cristal, planta 28**
**28046 Madrid (ES)**

# (54) MONO-P-TOLUENESULFONATE OF AXL KINASE INHIBITOR AND CRYSTAL FORM THEREOF

(57) The present invention provides a mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethyl phosphine oxide, and a hydrate and a crystal form thereof. The crystal form of p-toluenesulfonate has a good stability, is easy to process, and has a high solubility.

EP 4 438 613 A1

## Description

### TECHNICAL FIELD

The present invention belongs to the field of medical technology. The compound is an AXL kinase inhibitor, and specifically relates to p-toluenesulfonate of the AXL inhibitor and crystal form thereof.

### BACKGROUND

Receptor tyrosine kinases (RTKs) are multidomain transmembrane proteins that serve as sensors for extracellular ligands. Ligand-receptor binding induces receptor dimerization and activation of its intracellular kinase domain, which in turn leads to the recruitment, phosphorylation, and activation of multiple downstream signaling cascades (Robinson, DR et al., Oncogene, 19:5548-5557, 2000). To date, 58 RTKs have been identified in the human genome, which regulate a variety of cellular processes, including cell survival, growth, differentiation, proliferation, adhesion, and motility (Segaliny, AI et al., J. Bone Oncol, 4:1 -12, 2015).

AXL (also known as UFO, ARK and Tyro7) belongs to the TAM family of receptor tyrosine kinases, which also includes Mer and Tyro3. Among them, AXL and Tyro3 have the most similar gene structures, while AXL and Mer have the most similar amino acid sequences of tyrosine kinase domains. Like other receptor tyrosine kinases (RTKs), the structure of the TAM family consists of an extracellular domain, a transmembrane domain, and a conserved intracellular kinase domain. The extracellular domain of AXL has a unique structure that juxtaposes immunoglobulin and type III fibronectin repeating units and is reminiscent of that of neutral cell adhesion molecules. TAM family members have a common ligand-growth arrest specific protein 6 (Gas6), which can bind to all TAM receptor tyrosine kinases. After AXL binds to Gas6, it will lead to receptor dimerization and AXL autophosphorylation, thereby activating multiple downstream signal transduction pathways and participating in multiple processes of tumorigenesis (Linger, R. M et al., Ther. Targets, 14 (10), 1073-1090, 2010; Rescigno, J. et al., Oncogene, 6(10), 1909-1913, 1991).

AXL is widely expressed in normal human tissues, such as monocytes, macrophages, platelets, endothelial cells, cerebellum, heart, skeletal muscle, liver and kidney, etc. Among them, myocardium and skeletal muscle have the highest expression, and bone marrow CD34+ cells and stromal cells also have higher expression, normal lymphoid tissue has low expression (Wu YM, Robinson DR, Kung HJ, Cancer Res, 64 (20), 7311-7320, 2004; Hung BI et al., DNA Cell Biol, 22 (8), 533-540, 2003). In studies of many cancer cells, it has been found that the AXL gene is overexpressed or ectopically expressed in hematopoietic cells, stromal cells, and endothelial cells. The overexpression of AXL kinase is particularly prominent in various types of leukemias and most solid tumors. By inhibiting AXL receptor tyrosine kinase, the pro-survival signals of tumor cells can be reduced, the invasion ability of tumors can be blocked, and the sensitivity of targeted drug therapy and chemotherapy can be increased. Therefore, finding effective AXL inhibitors is an important direction in the current research and development of tumor-targeted medicaments.

### SUMMARY OF THE INVENTION

In one aspect, the present invention provides a mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or a hydrate thereof.

Further, the mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof, described herein, has the structure as shown in formula I:

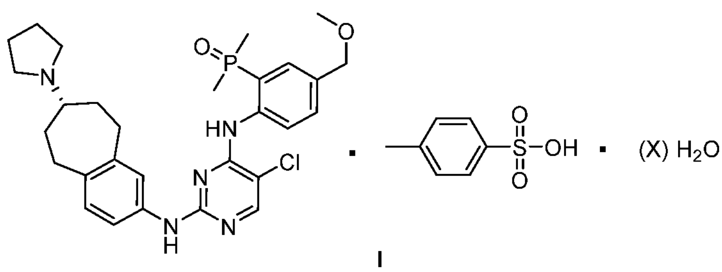

I

,

wherein X=0~2.

Further, X = 0~1 or 1.5.

Further, X is 0, 0.25, 0.5, 0.7, 1, 1.25, 1.5 or 1.75.

In some typical embodiments, X is 0.

In some typical embodiments, X is 0.7.

In some typical embodiments, X is 1.

In some typical embodiments, X is 1.5.

Further, the present invention provides a hydrate of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide.

In some typical embodiments, the present invention provides a monohydrate of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, the specific structure is shown in formula II,

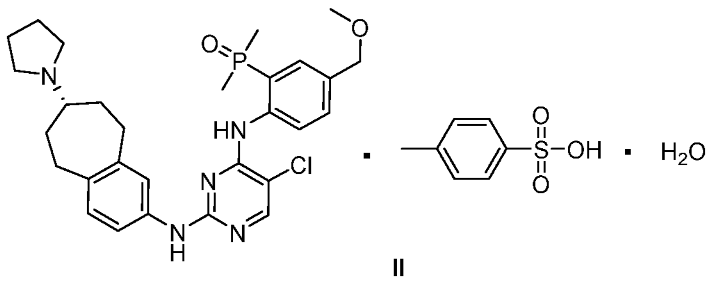

**II**

.

In some typical embodiments, the present invention provides a sesquihydrate of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, the specific structure is shown in formula III,

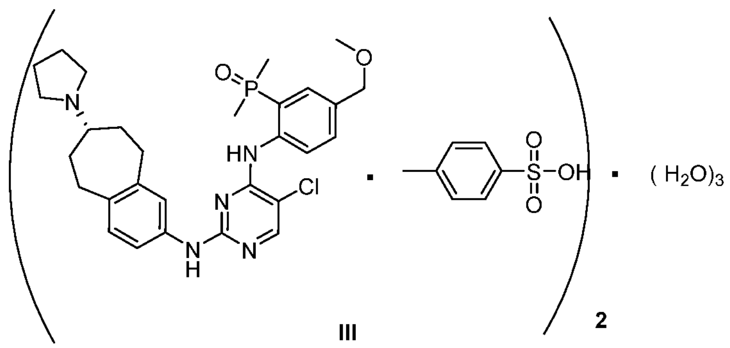

**III**

.

Further, the present invention provides a crystal form of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof.

Further, the present invention provides a crystal form of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof, the X-ray powder diffraction pattern has diffraction peaks at 2θ of 6.0°±0.2°, 6.3°±0.2°, 10.5° ±0.2 °, 13.2°±0.2° and 21.8° ±0.2°.

In some typical embodiments, the X-ray powder diffraction pattern of the crystal has diffraction peaks at 2θ of 6.0°±0.2°, 6.3°±0.2°, 10.5° ± 0.2°, 11.5° ± 0.2°, 13.2°±0.2°, 15.2°±0.2°, 18.0°±0.2°, 18.6°±0.2°, 21.8°±0.2° and 22.6°±0.2°.

In some typical embodiments, the X-ray powder diffraction pattern of the crystal has diffraction peaks at 2θ of 6.0°±0.2°, 6.3°±0.2°, 10.5°±0.2°, 11.5°±0.2°, 13.2°±0.2°, 15.2°±0.2°, 18.0°±0.2°, 18.6°±0.2°, 18.7°±0.2°, 19.4°±0.2°, 19.7°±0.2°, 21.8°±0.2°, 22.6°±0.2° and 29.4°±0.2°.

In some more typical embodiments, the X-ray powder diffraction pattern of the crystal has diffraction peaks at 2θ of 6.0°±0.2°, 6.3°±0.2°, 10.5°±0.2°, 11.5°±0.2°, 12.3°±0.2°, 12.4°±0.2°, 12.6°±0.2°, 13.2°±0.2°, 14.1°±0.2°, 15.2°±0.2°, 15.9°±0.2°, 16.7°±0.2°, 17.1°±0.2°, 18.0°±0.2°, 18.6°±0.2°, 18.7°±0.2°, 19.0°±0.2°, 19.4°±0.2°, 19.7°±0.2°, 21.1°±0.2°, 21.8°±0.2°, 22.6°±0.2°, 23.3°±0.2°, 23.7°±0.2°, 24.1°±0.2°, 24.4°±0.2°, 24.7°±0.2°, 25.1°±0.2°, 26.2°±0.2°, 26.6°±0.2°, 27.0°±0.2°, 27.7°±0.2°, 28.0°±0.2°, 28.3°±0.2°, 28.7°±0.2°, 28.8°±0.2°, 29.4°±0.2°, 30.2°±0.2° and 33.9°±0.2°.

In some embodiments, the crystal has an endothermic peak at an onset temperature of 195±5°C~205±5°C in a thermal analysis diagram measured by differential scanning calorimetry.

In some embodiments, the crystal has an endothermic peak at an onset temperature of 198±5°C~203±5°C in a thermal analysis diagram measured by differential scanning calorimetry.

In some embodiments, the crystal has an endothermic peak at an onset temperature of 200±5°C in a thermal analysis diagram measured by differential scanning calorimetry.

Further, the present invention provides a crystal form of the compound of formula I, wherein X=0~1, the X-ray powder diffraction pattern has diffraction peaks at 2θ of 6.0º±0.2º, 6.3º±0.2º, 10.5º±0.2º, 13.2º±0.2º and 21.8º ±0.2º.

In some typical embodiments, the X-ray powder diffraction pattern of the crystal has diffraction peaks at 2θ of 6.0º±0.2º, 6.3º±0.2º, 10.5º±0.2º, 11.5º±0.2º, 13.2º±0.2º, 15.2º±0.2º, 18.0º±0.2º, 18.6º±0.2º, 21.8º±0.2º and 22.6º±0.2º.

In some typical embodiments, the X-ray powder diffraction pattern of the crystal has diffraction peaks at 2θ of 6.0º±0.2º, 6.3º±0.2º, 10.5º±0.2º, 11.5º±0.2º, 13.2º ±0.2º, 15.2º±0.2º, 18.0º±0.2º, 18.6º±0.2º, 18.7º±0.2º, 19.4º±0.2º, 19.7º±0.2º, 21.8º±0.2º, 22.6º±0.2º and 29.4º±0.2º.

In some more typical embodiments, the X-ray powder diffraction pattern of the crystal has diffraction peaks at 2θ of 6.0º±0.2º, 6.3º±0.2º, 10.5º±0.2º, 11.5º±0.2º, 12.3º±0.2º, 12.4º±0.2º, 12.6º±0.2º, 13.2º±0.2º, 14.1º±0.2º, 15.2º±0.2º, 15.9º±0.2º, 16.7º±0.2º, 17.1º±0.2º, 18.0º±0.2º, 18.6º±0.2º, 18.7º±0.2º, 19.0º±0.2º, 19.4º±0.2º, 19.7º±0.2º, 21.1º±0.2º, 21.8º±0.2º, 22.6º±0.2º, 23.3º±0.2º, 23.7º±0.2º, 24.1º±0.2º, 24.4º±0.2º, 24.7º±0.2º, 25.1º±0.2º, 26.2º±0.2º, 26.6º±0.2º, 27.0º±0.2º, 27.7º±0.2º, 28.0º±0.2º, 28.3º±0.2º, 28.7º±0.2º, 28.8º±0.2º, 29.4º±0.2º, 30.2º±0.2º and 33.9º±0.2º.

In some embodiments, the crystal has an endothermic peak at an onset temperature of 195±5°C~205±5°C in a thermal analysis diagram measured by differential scanning calorimetry. In some embodiments, the crystal has an endothermic peak at an onset temperature of 198±5°C~203±5°C in a thermal analysis diagram measured by differential scanning calorimetry. In some more typical embodiments, the crystal has an endothermic peak at an onset temperature of 200°C±5°C in a thermal analysis diagram measured by differential scanning calorimetry.

Further, the present invention provides a crystal form B of mono-p-toluenesulfonate monohydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, the X-ray powder diffraction pattern has diffraction peaks at 2θ of 6.0º±0.2º, 6.3º±0.2º, 10.5º±0.2º, 13.2º±0.2ºand 21.8º±0.2º.

In some embodiments, the X-ray powder diffraction pattern of the crystal form B has diffraction peaks at 2θ of 6.0º±0.2º, 6.3º±0.2º, 10.5º±0.2º, 11.5º±0.2º, 13.2º±0.2º, 15.2º±0.2º, 18.0º±0.2º, 18.6º±0.2º, 21.8º±0.2º and 22.6º±0.2º.

In some embodiments, the X-ray powder diffraction pattern of the crystal form B has diffraction peaks at 2θ of 6.0º±0.2º, 6.3º±0.2º, 10.5º±0.2º, 11.5º±0.2º, 13.2º±0.2º, 15.2º±0.2º, 18.0º±0.2º, 18.6º±0.2º, 18.7º±0.2º, 19.4º±0.2º, 19.7º±0.2º, 21.8º±0.2º, 22.6º±0.2º and 29.4º±0.2.

In some embodiments, the X-ray powder diffraction pattern of the crystal form B has diffraction peaks at 2θ of 6.0º±0.2º, 6.3º±0.2º, 10.5º±0.2º, 11.5º±0.2º, 12.3º±0.2º, 12.4º±0.2º, 12.6º±0.2º, 13.2º±0.2º, 14.1º±0.2º, 15.2º±0.2º, 15.9º±0.2º, 16.7º±0.2º, 17.1º±0.2º, 18.0º±0.2º, 18.6º±0.2º, 18.7º±0.2º, 19.0º±0.2º, 19.4º±0.2º, 19.7º±0.2º, 21.1º±0.2º, 21.8º±0.2º, 22.6º±0.2º, 23.3º±0.2º, 23.7º±0.2º, 24.1º±0.2º, 24.4º±0.2º, 24.7º±0.2º, 25.1º±0.2º, 26.2º±0.2º, 26.6º±0.2º, 27.0º±0.2º, 27.7º±0.2º, 28.0º±0.2º, 28.3º±0.2º, 28.7º±0.2º, 28.8º±0.2º, 29.4º±0.2º, 30.2º±0.2º and 33.9º±0.2º. In some more typical embodiments, the X-ray powder diffraction of the crystal form B expressed in an angle of 2θ has the data described in the following table:

Table 1: X-ray powder diffraction pattern data of crystal form B

| 2θ (º) | Counts | $I/I_0$ (%) |
|---|---|---|
| 6.0 | 1772 | 100.0 |
| 6.3 | 1424 | 80.3 |
| 7.7 | 36 | 2.0 |
| 8.1 | 26 | 1.4 |
| 9.2 | 12 | 0.7 |
| 9.7 | 16 | 0.9 |
| 10.5 | 1448 | 81.7 |

(continued)

| 2θ (º) | Counts | $I/I_0$ (%) |
|---|---|---|
| 11.5 | 970 | 54.8 |
| 12.3 | 215 | 12.1 |
| 12.4 | 133 | 7.5 |
| 12.6 | 169 | 9.5 |
| 13.2 | 1033 | 58.3 |
| 13.6 | 53 | 3.0 |
| 14.1 | 190 | 10.7 |
| 15.2 | 804 | 45.4 |
| 15.9 | 281 | 15.9 |
| 16.7 | 323 | 18.2 |
| 17.1 | 292 | 16.5 |
| 18.0 | 974 | 54.9 |
| 18.6 | 947 | 53.4 |
| 18.7 | 449 | 25.3 |
| 19.0 | 354 | 20.0 |
| 19.4 | 764 | 43.1 |
| 19.7 | 487 | 27.5 |
| 20.6 | 50 | 2.8 |
| 21.1 | 239 | 13.5 |
| 21.8 | 1152 | 65.0 |
| 22.6 | 844 | 47.6 |
| 23.4 | 106 | 6.0 |
| 23.7 | 232 | 13.1 |
| 24.1 | 184 | 10.4 |
| 24.4 | 181 | 10.2 |
| 24.7 | 274 | 15.5 |
| 25.1 | 227 | 12.8 |
| 25.6 | 71 | 4.0 |
| 26.3 | 177 | 10.0 |
| 26.6 | 223 | 12.6 |
| 27.0 | 371 | 20.9 |
| 27.7 | 249 | 14.1 |
| 28.0 | 143 | 8.1 |
| 28.3 | 226 | 12.8 |
| 28.7 | 109 | 6.2 |
| 28.8 | 303 | 17.1 |
| 29.4 | 459 | 25.9 |
| 30.2 | 281 | 15.8 |

(continued)

| 2θ (º) | Counts | $I/I_0$ (%) |
|---|---|---|
| 32.0 | 81 | 4.5 |
| 33.1 | 68 | 3.9 |
| 33.9 | 152 | 8.6 |
| 35.1 | 58 | 3.3 |
| 35.7 | 44 | 2.5 |

In some embodiments, the X-ray powder diffraction of the crystal form B expressed in an angle of 2θ has a pattern as shown in Figure 1.

In some embodiments, the crystal form B has an endothermic peak at an onset temperature of 85±5°C to 95±5°C and an endothermic peak at an onset temperature of 195±5°C~205±5°C in a thermal analysis diagram measured by differential scanning calorimetry.

In some embodiments, the crystal form B has an endothermic peak at an onset temperature of 88±5°C to 93±5°C and an endothermic peak at an onset temperature of 198±5°C to 203±5°C in a thermal analysis diagram measured by differential scanning calorimetry.

In some embodiments, the crystal form B has an endothermic peak at an onset temperature of 90±5°C and an endothermic peak at an onset temperature of 200±5°C in a thermal analysis diagram measured by differential scanning calorimetry.

In some embodiments, the crystal form B has a pattern as shown in Figure 2 in a thermal analysis diagram measured by differential scanning calorimetry.

In some embodiments, a spectrum of the crystal form B that is obtained by attenuated total reflectance Fourier transform infrared spectroscopy has the following absorption bands expressed in reciprocals of wavelengths ($cm^{-1}$): 432±2, 471±2, 552±2, 570±2, 711±2, 747±2, 779±2, 818±2, 836±2, 863±2, 932±2, 966±2, 1295±2, 1318±2, 2635±2, 2721±2, 2927±2, 3005±2, 3110±2, 3185±2, 3256±2 and 3556±2.

In some embodiments, a spectrum of the crystal form B that is obtained by attenuated total reflectance Fourier transform infrared spectroscopy has the following absorption bands expressed in reciprocals of wavelengths ($cm^{-1}$): 432±2, 471±2, 497±2, 552±2, 570±2, 681±2, 711±2, 747±2, 779±2, 818±2, 836±2, 863±2, 932±2, 966±2, 1008±2, 1030±2, 1096±2, 1121±2, 1159±2, 1229±2, 1295±2, 1318±2, 1374±2, 1413±2, 1456±2, 1512±2, 1527±2, 1571±2, 1609±2, 2635±2, 2721±2, 2927±2, 3005±2, 3110±2, 3185±2, 3256±2 and 3556±2.

In some embodiments, a spectrum of the crystal form B that is obtained by Fourier transform Raman spectroscopy has the following absorption bands expressed in reciprocals of wavelengths ($cm^{-1}$): 1609±2, 1572±2, 1553±2, 1535±2, 1508±2, 1494±2, 1476±2, 1457±2, 1420±2, 1374±2, 1344±2, 1331±2, 1285±2, 1262±2, 1228±2, 1213±2, 1173±2, 1147±2, 1135±2, 1099±2, 1059±2, 1029±2, 1115±2, 1006±2, 970±2, 965±2, 819±2, 799±2, 744±2, 733±2, 678±2, 658±2, 634±2, 613±2, 570±2, 534±2, 497±2, 471±2, 448±2, 425±2, 393±2, 369±2, 313±2, 287±2, 262±2, 241±2, 217±2, 177±2, 156±2, 104±2 and 82±2.

In some embodiments, the crystal form B lost 2.4% of its weight in the temperature range of 25°C-150°C.

In some embodiments, the crystal form B has a TGA pattern as shown in Figure 3.

In some more exemplary embodiments, the crystal form B has single crystal parameters and structural data as described in the following table:

Table 2: crystal parameters and structural data of crystal form B

| Analytical data | |
|---|---|
| Molecular formula | $C_{29}H_{38}ClN_5O_2P{\cdot}C_7H_7O_3S{\cdot}H_2O$ |
| Molecular weight | 744.28 |
| Crystal system | Orthorhombic |
| Space group | $P2_12_12_1$ |
| Cell parameters | a=7.9891 (3)Å, α=90° |
| | b=15.7648(7)Å, β=90° |
| | c=29.3381 (14)Å, γ=90° |

(continued)

| Analytical data | |
|---|---|
| Crystal axis ratio | a/b=0.5068, b/c=0.5367, c/a=3.6723 |
| Z | 4 |
| Unit cell volume | 3695.0(3)Å$^3$ |
| Theoretical density | 1.338Mg/m$^3$ |
| $R_1$ | 0.050 |
| $WR_2$ | 0.114 |
| GOOF=S | 1.03 |
| $R_{(int)}$ | 0.082 |
| Flackparameter | 0.019(19) |

Further, the present invention provides a crystal form C of mono-p-toluenesulfonate sesquihydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, the soecific structure is shown in formula III:

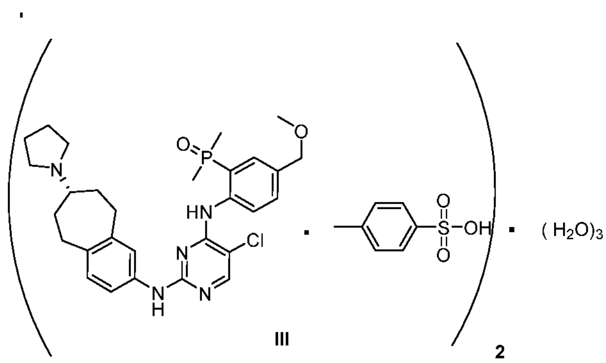

III

The X-ray powder diffraction pattern has diffraction peaks at 2θ of 13.4º±0.2º, 18.4º±0.2º, 19.3º±0.2º, 19.8º±0.2º and 21.8º±0.2º.

In some embodiments, the X-ray powder diffraction pattern of the crystal form C has diffraction peaks at 2θ of 12.1º±0.2º, 13.4º±0.2º, 15.0º±0.2º, 16.9º±0.2º, 18.4º±0.2º, 19.3º±0.2º, 19.8º±0.2º, 21.8º±0.2º, 23.6º±0.2º and 24.3º±0.2º.

In some embodiments, the X-ray powder diffraction pattern of the crystal form C has diffraction peaks at 2θ of 6º±0.2º, 10.8º±0.2º, 12.1º±0.2º, 13.4º±0.2º, 15.0º±0.2º, 16.9º±0.2º, 18.4º±0.2º, 19.0º±0.2º, 19.3º±0.2º, 19.8º±0.2º, 20.9º±0.2º, 21.8º±0.2º, 23.2º±0.2º, 23.6º±0.2º, 24.3º±0.2º, 25.5º±0.2º and 26.6º±0.2º.

In some more exemplary embodiments, the 2θ of the X-ray powder diffraction pattern of the crystal form C is detailed in the following table:

Table 3: X-ray powder diffraction pattern data of crystal form C

| 2θ (º) | Counts | $I/I_0$ (%) |
|---|---|---|
| 6.0 | 105 | 22.2 |
| 10.8 | 60 | 12.8 |
| 12.1 | 227 | 48.2 |
| 13.4 | 322 | 68.5 |
| 15.0 | 220 | 46.7 |
| 16.9 | 221 | 46.9 |

(continued)

| 2θ (º) | Counts | $I/I_0$ (%) |
|---|---|---|
| 18.4 | 471 | 100.0 |
| 19.0 | 176 | 37.5 |
| 19.3 | 238 | 50.5 |
| 19.8 | 306 | 65.0 |
| 20.9 | 154 | 32.8 |
| 21.8 | 310 | 65.9 |
| 23.2 | 172 | 36.6 |
| 23.6 | 231 | 49.1 |
| 24.3 | 213 | 45.2 |
| 25.5 | 62 | 13.1 |
| 26.6 | 103 | 21.8 |
| 27.7 | 38 | 8.0 |
| 29.7 | 93 | 19.7 |

In some embodiments, X-ray powder diffraction of the crystal form C expressed in a 2θ angle has a pattern as shown in Figure 7.

In some more exemplary embodiments, the crystal form C has single crystal parameters and structural data as described in the following table:

Table 4: crystal parameters and structure data of crystal form C

| Analytical data | |
|---|---|
| Molecular formula | $2(C_7H_7O_3S)\cdot 2(C_{29}H_{38}ClN_5O_2P)\cdot 3(H_2O)$ |
| Molecular weight | 1506.54 |
| Crystal system | Orthorhombic |
| Space group | $P2_12_12_1$ |
| Cell parameters | a=8.4802(8)Å, α=90° |
| | b=15.1272(14)Å, β=90° |
| | c=29.429(3)Å, γ=90° |
| Crystal axis ratio | a/b=0.5606, b/c=0.5140, c/a=3.4703 |
| Z | 2 |
| Unit cell volume | 3775.2(7)$Å^3$ |
| Theoretical density | 1.325$Mg/m^3$ |
| $R_1$ | 0.053 |
| $WR_2$ | 0.114 |
| GOOF=S | 1.06 |
| $R_{(int)}$ | 0.041 |
| Flackparameter | 0.029(12) |

In another aspect, the present invention provides a crystal form A of the compound of formula IV, the X-ray

powder diffraction pattern has diffraction peaks at 2θ of 7.6º±0.2º, 10.2º±0.2º, 17.6 º±0.2º, 20.3º±0.2º and 20.9º±0.2º.

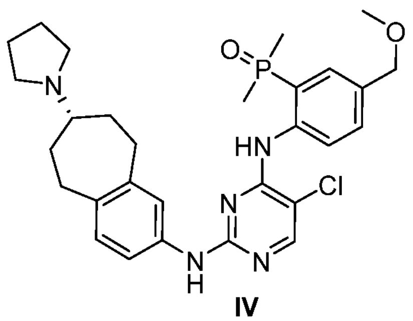

**IV**

In some embodiments, the X-ray powder diffraction pattern of the crystal form A has diffraction peaks at 2θ of 4.1º±0.2º, 7.6º±0.2º, 10.2º±0.2º, 12.6º±0.2º, 13.0º±0.2º, 17.6º±0.2º, 19.7º±0.2º, 20.3º±0.2º, 20.9º±0.2º and 22.2º±0.2º.

In some embodiments, the X-ray powder diffraction pattern of the crystal form A has diffraction peaks at 2θ of 4.1º±0.2º, 5.6º±0.2º, 7.6º±0.2º, 10.2º±0.2º, 10.9º±0.2º, 12.6º±0.2º, 13.0º±0.2º, 15.2º±0.2º, 17.6º±0.2º, 19.7º±0.2º, 20.3 º±0.2º, 20.9º±0.2º, 22.2º±0.2º, 23.2º±0.2º, 24.6º±0.2º, 27.0º±0.2º, 28.8º±0.2º, 37.0º±0.2º and 37.7º±0.2º.

In some more exemplary embodiments, the 2θ of the X-ray powder diffraction pattern of the crystal form A is detailed in the following table:

Table 5: X-ray powder diffraction pattern data of crystal form A

| 2θ (º) | Counts | $I/I_0$ (%) |
|---|---|---|
| 4.1 | 45 | 15.4 |
| 5.6 | 42 | 14.3 |
| 7.6 | 109 | 36.9 |
| 10.2 | 107 | 36.4 |
| 10.9 | 13 | 4.4 |
| 12.6 | 59 | 20.1 |
| 13.0 | 44 | 14.8 |
| 15.2 | 18 | 6.2 |
| 17.6 | 246 | 83.4 |
| 19.7 | 85 | 28.9 |
| 20.3 | 184 | 62.5 |
| 20.9 | 294 | 100.0 |
| 22.2 | 60 | 20.5 |
| 23.2 | 22 | 7.5 |
| 24.6 | 19 | 6.6 |
| 27.0 | 40 | 13.6 |
| 28.8 | 14 | 4.6 |
| 37.0 | 6 | 2.1 |
| 37.7 | 12 | 4.1 |

In some more exemplary embodiments, X-ray powder diffraction of the crystal form A expressed in 2θ angle

has a pattern as shown in Figure 9.

In another aspect, the present invention provides methods for preparing mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, or pharmaceutically acceptable hydrate thereof, comprising the step of salifying the compound of formula IV with p-toluenesulfonic acid.

In another aspect, the present invention provides methods for preparing the compound of formula I or crystal form thereof, comprising the step of salifying the compound of formula IV with p-toluenesulfonic acid.

In some embodiments, the crystal form of the compound of formula I can be prepared by mixing and stirring, gas-solid diffusion, solvent evaporation, or precipitation at reduced temperature.

In another aspect, the present invention provides methods for preparing the crystal form B by reacting the compound of formula IV with p-toluenesulfonic acid monohydrate in an alcoholic solvent and crystallizing in an anti-solvent.

In some embodiments, the alcohol solvent may be an alcohol solvent or a mixture of alcohol and water, the alcohol solvent is selected from one of methanol, ethanol and isopropanol, preferably ethanol or isopropanol.

In some embodiments, the anti-solvent is selected from ester solvent, ketone solvent or ether solvent, wherein the ketone solvent is acetone, 2-butanone or methyl isobutyl ketone, preferably acetone; the ether solvent is methyl tert-butyl ether or 1,4-dioxane, preferably methyl tert-butyl ether; and the ester solvent is selected from ethyl acetate, butyl acetate or isopropyl acetate, preferably isopropyl acetate.

In another aspect, the present invention provides a pharmaceutical composition comprising mono-p-toluenesulfonate of (S)-(2-((5-chloro-2-((7-(pyrrolidin-1 -yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof.

In another aspect, the present invention provides a pharmaceutical composition comprising the compound of formula I.

In another aspect, the present invention provides a pharmaceutical composition comprising the compound of formula II.

In another aspect, the present invention provides a pharmaceutical composition comprising the compound of formula III.

In some embodiments, the pharmaceutical composition further comprises one or more pharmaceutically acceptable carriers.

In some embodiments, the pharmaceutical composition is solid pharmaceutical preparation suitable for oral administration, preferably tablets or capsules.

In another aspect, the present invention provides a crystal form composition comprising the crystal form A, wherein the crystal form A comprises more than 50% by weight of the weight of the crystal form composition; preferably more than 80% by weight; further preferably more than 90% by weight; further preferably more than 95% by weight; most preferably more than 98% by weight.

In another aspect, the present invention also provides a pharmaceutical composition comprises the crystal form A or crystal form composition thereof.

In some embodiments, the pharmaceutical composition further comprises one or more pharmaceutically acceptable carriers.

In some embodiments, the pharmaceutical composition is a solid pharmaceutical preparation suitable for oral administration, preferably tablets or capsules.

In another aspect, the present invention provides a crystal form composition comprising the crystal form B, wherein the crystal form B is more than 50% by weight of the weight of the crystal form composition; preferably more than 80% by weight; further preferably more than 90% by weight; further preferably more than 95% by weight; most preferably more than 98% by weight. In another aspect, the present invention also provides a pharmaceutical composition comprising the crystal form B or crystal form composition.

In some embodiments, the pharmaceutical composition further comprises one or more pharmaceutically acceptable carriers.

In some embodiments, the pharmaceutical composition is a solid pharmaceutical preparation suitable for oral administration, preferably tablets or capsules.

In another aspect, the present invention provides a crystal form composition comprising the crystal form C, wherein the crystal form C comprises more than 50% by weight of the weight of the crystal form composition; preferably more than 80% by weight; further preferably more than 90% by weight; further preferably more than 95% by weight; most preferably more than 98% by weight.

In another aspect, the present invention also provides a pharmaceutical composition comprising the crystal form C or crystal form composition thereof.

In some embodiments, the pharmaceutical composition further comprises one or more pharmaceutically acceptable carriers.

In some embodiments, the pharmaceutical composition is a solid pharmaceutical preparation suitable for oral administration, preferably tablets or capsules.

In another aspect, the present invention also provides the compound of formula I or pharmaceutical composition thereof for use as medicaments.

In another aspect, the present invention also provides the compound of formula II or pharmaceutical composition thereof for use as medicaments.

In another aspect, the present invention also provides the compound of formula III or pharmaceutical composition thereof for use as medicaments.

In another aspect, the present invention also provides the crystal form A, crystal form composition thereof or pharmaceutical composition thereof for use as medicaments.

In another aspect, the present invention also provides the crystal form B, crystal form composition thereof or pharmaceutical composition thereof for use as medicaments.

In another aspect, the present invention also provides the crystal form C, crystal form composition thereof or pharmaceutical composition thereof for use as medicaments.

In another aspect, the present invention also provides the use of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, hydrate thereof and pharmaceutical composition thereof in the preparation of medicaments for the prevention and/or treatment of AXL kinase-mediated diseases or disease states.

In another aspect, the present invention also provides the use of the compound of formula I or pharmaceutical composition thereof in the preparation of medicaments for the prevention and/or treatment of AXL kinase-mediated diseases or disease states.

In another aspect, the present invention also provides the use of the compound of formula II or pharmaceutical composition thereof in the preparation of medicaments for the prevention and/or treatment of AXL kinase-mediated diseases or disease states.

In another aspect, the present invention also provides the use of the compound of formula III or pharmaceutical composition thereof in the preparation of medicaments for the prevention and/or treatment of AXL kinase-mediated diseases or disease states.

In another aspect, the present invention also provides the use of the crystal form A or pharmaceutical composition thereof in the preparation of medicaments for the prevention and/or treatment of AXL kinase-mediated diseases or disease states.

In another aspect, the present invention also provides the use of the crystal form B or pharmaceutical composition thereof in the preparation of medicaments for the prevention and/or treatment of AXL kinase-mediated diseases or disease states.

In another aspect, the present invention also provides the use of the crystal form C or pharmaceutical composition thereof in the preparation of medicaments for the prevention and/or treatment of AXL kinase-mediated diseases or disease states.

In another aspect, the present invention also provides the use of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof in the preparation of medicaments in the prevention and/or treatment of an AXL kinase-mediated disease or disease state.

In another aspect, the present invention also provides the use of the compound of formula I in the preparation of medicaments for the prevention and/or treatment of AXL kinase-mediated diseases or disease states.

In another aspect, the present invention also provides the use of the compound of formula II in the preparation of medicaments for the prevention and/or treatment of AXL kinase-mediated diseases or disease states.

In another aspect, the present invention also provides the use of the compound of formula III in the preparation of medicaments for the prevention and/or treatment of AXL kinase-mediated diseases or disease states.

In another aspect, the present invention also provides the use of the crystal form composition in the preparation of medicaments for the prevention and/or treatment of AXL kinase-mediated diseases or disease states.

In another aspect, the present invention also provides the use of the crystal form A, crystal form composition thereof or pharmaceutical composition thereof for the prevention and/or treatment of AXL kinase-mediated diseases or disease states.

In another aspect, the present invention also provides the use of the crystal form B, crystal form composition thereof or pharmaceutical composition thereof for the prevention and/or treatment of AXL kinase-mediated diseases or disease states.

In another aspect, the present invention also provides the use of the crystal form C, crystal form composition thereof or pharmaceutical composition thereof for the prevention and/or treatment of AXL kinase-mediated diseases or disease states.

In another aspect, the present invention also provides methods for preventing and/or treating AXL kinase-mediated diseases or disease states comprising administering to an individual in need thereof the mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, hydrate thereof, or pharmaceutical composition thereof.

In another aspect, the present invention also provides methods for preventing and/or treating AXL kinase-mediated diseases or disease states, it comprises administering to an individual in need thereof the compound of formula I of the present invention or a pharmaceutical composition thereof.

In another aspect, the present invention also provides methods for preventing and/or treating AXL kinase-mediated diseases or disease states, it comprises administering to an individual in need thereof the compound of formula II of the present invention or a pharmaceutical composition thereof.

In another aspect, the present invention also provides methods for preventing and/or treating AXL kinase-mediated diseases or disease states, it comprises administering to an individual in need thereof the compound of formula III of the present invention or pharmaceutical composition thereof.

In another aspect, the present invention also provides methods for preventing and/or treating AXL kinase-mediated diseases or disease states, it comprises administering the crystal form composition of the present invention to an individual in need thereof.

In another aspect, the present invention also provides methods for preventing and/or treating AXL kinase-mediated diseases or disease states, it comprises administering the crystal form A of the present invention or a pharmaceutical composition thereof to an individual in need thereof.

In another aspect, the present invention also provides methods for preventing and/or treating AXL kinase-mediated diseases or disease states, it comprises administering the crystal form B of the present invention or a pharmaceutical composition thereof to an individual in need thereof.

In another aspect, the present invention also provides methods for preventing and/or treating AXL kinase-mediated diseases or disease states, it comprises administering the crystal form C of the present invention or a pharmaceutical composition thereof to an individual in need thereof.

In another aspect, the present invention also provides mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof for use in the prevention and/or treatment of AXL kinase-mediated diseases or disease states.

In another aspect, the present invention also provides the compound of formula I of the present invention for use in the prevention and/or treatment of AXL kinase-mediated diseases or disease states.

In another aspect, the present invention also provides the compound of formula II of the present invention for use in the prevention and/or treatment of AXL kinase-mediated diseases or disease states.

In another aspect, the present invention also provides the compound of formula III of the present invention for use in the prevention and/or treatment of AXL kinase-mediated diseases or disease states.

In another aspect, the present invention also provides the crystal form composition of the present invention for use in the prevention and/or treatment of AXL kinase-mediated diseases or disease states.

In another aspect, the present invention also provides the crystal form A of the present invention or pharmaceutical composition thereof for use in the prevention and/or treatment of AXL kinase-mediated diseases or disease states.

In another aspect, the present invention also provides the crystal form B of the present invention or pharmaceutical composition thereof for use in the prevention and/or treatment of AXL kinase-mediated diseases or disease states.

In another aspect, the present invention also provides the crystal form C of the present invention or pharmaceutical composition thereof for use in the prevention and/or treatment of AXL kinase-mediated diseases or disease states.

In some embodiments, the AXL kinase-mediated disease or disease state is cancer.

In some typical embodiments, the cancers are associated with hematologic neoplasms.

The mono-p-toluenesulfonate of the compound of formula IV prepared by the present invention, hydrate thereof and the crystal form of the hydrate have good stability, solving the problem of the instability of the free base (compound of formula IV) at high temperature, high humidity, and under the conditions of illumination, and the crystal form obtained by the preparation method has the advantages of being stable, easy to be processed, and has high solubility.

## Relevant definitions

Unless otherwise specified, the following terms used in the description and claims have the following meanings: The term "pharmaceutically acceptable carrier" refers to those carriers that have no obvious irritating effect on the body and do not impair the biological activity and performance of the active compound. Including but not limited to any diluent, disintegrant, binder, glidant, and wetting agent approved by the National Medical Products Administration for use on humans or animals.

The "X-ray powder diffraction pattern spectrum" in the present invention is obtained by using Cu-K$\alpha$ radiation measurement.

"2θ" or "2θ angle" in the present invention refers to the diffraction angle, θ is the Bragg angle, and the unit is ° or degree; the error range of each characteristic peak 2θ is ±0.20°.

It should be noted that in X-ray powder diffraction spectroscopy (XRPD), the diffraction spectra obtained from crystalline compounds tend to be characteristic for a particular crystallization, where the relative intensities of the bands (especially at low angles) may vary due to the dominant orientation effect resulting from differences in crystallization conditions, particle size, and other measurement conditions. Therefore, the relative intensities of the diffraction peaks are not characteristic of the crystallization in question. It is the relative positions of the peaks rather than their relative intensities that should be taken into account when determining whether the crystallization is the same as a known one. In addition, it is well known in the field of crystallography that there may be a slight error in the position of the peaks for any given crystallization. For example, the position of the peaks can shift due to changes in temperature when analyzing the sample, sample movement, or instrument calibration, and the error in the determination of the 2θ value is sometimes about ±0.2°. Therefore, this error should be taken into account when determining each crystal structure. In the XRPD spectrum, the 2θ angle or crystal plane distance d is usually used to indicate the peak position, and the two have a simple conversion relationship: $d = \lambda/2\sin\theta$, wherein d represents the crystal plane distance, λ represents the wavelength of the incident X-rays, and θ is the diffraction angle. For the same crystallization of the same compound, the peak positions of its XRPD spectra have similarity in the whole, and the relative intensity error may be larger. It should also be noted that in the identification of mixtures, some diffraction lines are missing due to factors such as decreasing content, etc. In this case, it is not necessary to rely on all the spectral bands observed in the high-purity specimen, and even a single band may be characteristic for a given crystallization.

Differential Scanning Calorimetry (DSC) determines the transition temperature when a crystal absorbs or releases heat as a result of changes in its crystal structure or as a result of crystal melting. For the same crystalline form of the same compound, the thermal transition temperature and melting point are typically within about 5°C of each other in successive analyses, and usually within about 3°C of each other. When describing a compound as having a given DSC peak or melting point, this refers to the DSC peak or melting point ± 5°C. DSC provides an aid in recognizing different crystal forms. Different crystal forms can be recognized based on their different transition temperature characteristics. It should be noted that for mixtures, the DSC peak or melting point may fluctuate over a wider range. In addition, the melting temperature is related to the rate of heating, since decomposition occurs during the melting process.

Thermogravimetric analysis (TGA) refers to a thermal analysis technique that measures the relationship between the mass of a sample to be measured and the change in temperature at a programmed temperature. When the substance to be measured is sublimated or vaporized during the heating process, resulting in the decomposition into gas or the loss of crystalline water of crystallization, causing a change in the mass of the substance to be measured. In this case, the thermogravimetric curve is not straight but decreases. By analyzing the thermogravimetric curve, it is possible to know at what temperature the substance to be measured changes, and according to the weight lost, it is possible to calculate the amount of substance lost.

When referring to, for example, an XRPD pattern, a DSC pattern or a TGA pattern, the term "as shown" includes patterns that are not necessarily the same as those depicted herein, but which fall within the limits of experimental error when considered by one skilled in the art.

As used herein, the term "hydrate" is a specific solvent compound in which the solvent is water, and examples of hydrates include hemihydrate, monohydrate, sesquihydrate, dihydrate, and the like.

Different crystalline forms of a particular substance, such as a salt of the present invention, may include both an anhydrous form of the substance and a hydrated form of the substance, wherein each of the anhydrous form and the hydrated form are distinguishable from each other by a different XRPD image and thus by representing a different crystal lattice. In some examples, a single crystalline form (e.g., identified by a separate XRPD image) may have a variable water content or solvent content, wherein the lattice remains essentially unchanged (as represented by the XRPD image) except for changes in composition relative to water and/or solvent.

Unless otherwise specified, the abbreviations of the present invention have the following meanings:

M: mol/L
mM: mmol/L
nM: nmol/L
Boc: tert-butoxycarbonyl
$^1$HNMR: Hydrogen Nuclear Magnetic Resonance Spectrum
MS(ESI+): Mass Spectrometry
DMSO- $d_6$: deuterated dimethyl sulfoxide
$CDCl_3$: deuterated chloroform
DTT: dithiothreitol
SEB: SupplementedEnzymaticBuffer (supplemented enzyme buffer)
IMDM ( Iscove'sModifiedDulbecco'sMedium): Iscove 's modified Dulbecco 's medium .

Room temperature: 25°C.

## Brief description of the drawings

In order to more clearly illustrate the technical solutions of the embodiments and prior art of the present invention, the following is a brief introduction to the embodiments and the prior art need to use the accompanying drawings, it is obvious that the following description of the accompanying drawings is only some of the embodiments of the present invention, for the person of ordinary skill in the field, according to these drawings, there are also other attached drawings can also be obtained.

Figure 1 is the X-ray powder diffraction (XRPD) spectrum of the crystal form B in Example 3.
Figure 2 is a differential scanning calorimetry (DSC) spectrum of the crystal form B in Example 3.
Figure 3 is the thermogravimetric (TGA) spectrum of the crystal form B in Example 3.
Figure 4 is the FT-IR (FT-IR) spectrum of the crystal form B in Example 3.
Figure 5 is the FT-Raman spectrum of the crystal form B in Example 3.
Figure 6 is the dynamic moisture adsorption (DVS) spectrum of the crystal form B in Example 3.
Figure 7 is the X-ray powder diffraction (XRPD) spectrum of the crystal form C in Example 6.
Figure 8 is the X-ray powder diffraction (XRPD) spectrum of the crystal form A in Example 8.
Figure 9 is the X-ray powder diffraction (XRPD) spectrum of the mesylate salt of the compound IV in Example 10.
Figure 10 is the X-ray powder diffraction (XRPD) spectrum of the monohydrochloride of the compound of formula IV in Example 10.
Figure 11 is the X-ray powder diffraction (XRPD) spectrum of the dihydrochloride of the compound of formula IV in Example 10.
Figure 12 is the X-ray powder diffraction (XRPD) spectrum of the phosphate of the compound of formula IV in Example 10.
Figure 13 is the X-ray powder diffraction (XRPD) spectrum of the hippurate of the compound of formula IV in Example 10.
Figure 14 is the X-ray powder diffraction (XRPD) spectrum of the sulfate of the compound of formula IV in Example 10.
Figure 15 is the X-ray powder diffraction (XRPD) spectrum of the hydrobromide salt of the compound of formula IV in Example 10.
Figure 16 is the X-ray powder diffraction (XRPD) spectrum of the benzene sulfonate salt of the compound of formula IV in Example 10.
Figure 17 is the X-ray powder diffraction (XRPD) spectrum of the oxalate salt of the compound of formula IV in Example 10.
Figure 18 is the X-ray powder diffraction (XRPD) spectrum of the fumarate salt of the compound of formula IV in Example 10.
Figure 19 is the X-ray powder diffraction (XRPD) spectrum of the citrate salt of the compound of formula IV in Example 10.
Figure 20 is an X-ray powder diffraction (XRPD) spectrum of the compound of formula I prepared in Example 11.

## Detailed description

The present invention is described in more detail below by means of examples. However, these specific descriptions are only used to illustrate the technical solutions of the present invention and do not constitute any limitation on the present invention.
The test conditions for each instrument are as follows:

(1) X-ray Powder Diffraction (XRPD)

Instrument model: Bruker D2 Phaser2$^{nd}$

| Reflective Mode | |
|---|---|
| X-ray | Cu,kα,Kα1(Å):1.540598, Kα2(Å):1.544426 Kα2/Kα1 intensity ratio: 0.50 |
| X-ray tube settings | 30kV, 10mA |
| Detector | 1D-LYNXEYE |
| Sola slit | 0.6mm |
| Sample rotation time (seconds) | 15 |
| Scan range (º2θ) | 3-40º |
| Scan step size (º2θ) | 0.043 |
| Scan time (seconds) | 150 |

(2) Thermogravimetric Analyzer (TGA)

Instrument model: TA Instruments TGA 25
Purge gas: nitrogen
Heating rate: 10ºC/min
Heating range: room temperature -300ºC
Method: placed the sample on an aluminum plate, then placed the aluminum plate on a platinum plate, and heated it from room temperature to the set temperature at a speed of 10ºC/min in an open nitrogen atmosphere.

(3) Differential Scanning Calorimeter (DSC)

Instrument model: TA Instruments DSC 25
Purge gas: nitrogen.
Heating rate: 10ºC/min
Heating range: 20-300ºC
Method: placed the sample on an aluminum plate, capped and heated the sample from 20ºC to the set temperature under nitrogen atmosphere at a rate of 10ºC/min.

(4) Fourier Transform Infrared Spectroscopy (FT-IR)

Instrument model: Thermo Fourier transform infrared spectrometer ID1-summit
Instrument calibration: polystyrene film
Test conditions: KBr tableting method

(5) Fourier Transform Raman Spectroscopy (FT-Raman)

Instrument model: Nicoret Fourier transform Raman spectrometer DXR780
Exposure time: 20s
Number of exposures: 10 times
Light source: 780 nm
Slit: 400 lines/mm
Laser intensity: 14 mW
Scanning range: 50 cm-1-3000 cm-1

(6) Dynamic Vapor Sorption (DVS)

Instrument model: Surface Measurement System(SMS)-DVS Intrinsic

| DVS | |
|---|---|
| Temperature | 25ºC |
| Sample quantity | 10-20mg |
| Protective gas and flow rate | $N_2$, 200mL/min |
| dm/dt | 0.002%/min |
| Minimum dm/dt balancing time | 10min |
| Maximum balancing time | 180min |
| Humidity range | Indoor humidity-95%RH-0%RH-95%RH |
| Humidity gradient | 10%(90%RH-0%RH-90%RH) |

The specific instrument setting parameters are as follows:

(7) Single crystal diffractometer and its parameter information:

Single Crystal X-ray Diffraction (SCXRD)
Instrument model: Bruker D8 Venture
Light source: Cu-Kα, λ=1.54Å
Detector: CMOS area detector
Resolution: 0.8Å
X-ray tube settings: Tube voltage 50KV, tube current 1.2mA
Exposure time: 50s
Test temperature: 170(2)K

(8) Analysis conditions of HPLC

| | | | |
|---|---|---|---|
| Equipment | | | High performance liquid chromatography (PDA detector) |
| Column | | | TitankC18 (4.6mm × 150mm, 3μm) or a chromatography column of |
| | equivalent performance | | |
| Wavelength (nm) | 280nm | Flow rate(ml/min) | 1.0 |
| Column temperature (°C) | 30 | Detection time (min) | 60 |
| Injection volume (μL) | 10 | Elution method | gradient elution |
| Mobile phase | A | 0.01 mol/L potassium dihydrogen phosphate buffer (1.36 g potassium dihydrogen phosphate was dissolved in 1L of water and pH was adjusted to 7.0 with potassium hydroxide solution) | |
| | B | Acetonitrile | |

(continued)

| | Time (min) | Mobile phase A (%) | Mobile phase B (%) |
|---|---|---|---|
| Run method | 0 | 85 | 15 |
| | 10 | 70 | 30 |
| | 15 | 70 | 30 |
| | 25 | 20 | 80 |
| | 30 | 20 | 80 |
| | 31 | 85 | 15 |
| | 40 | 85 | 15 |

**Example 1 Preparation of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide**

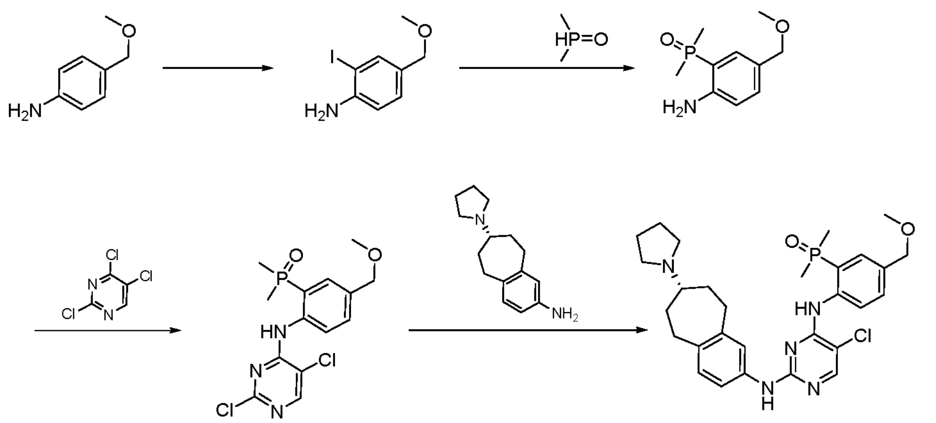

a) 2-Iodo-4-(methoxymethyl)aniline

4-(Methoxymethyl)aniline (9g), iodine (16.65g) and sodium bicarbonate (16.53g) were added to a solution of dichloromethane (261mL)/water (135mL), and the reaction solution was stirred at 22°C for 16h. The reaction solution was quenched with saturated sodium thiosulfate (10mL) at room temperature. The resulting mixture was extracted with dichloromethane (3x100mL), then the combined organic layers were washed with saturated aqueous sodium chloride solution (1×100mL) and dried over anhydrous sodium sulfate. After filtration, the filtrate was concentrated under reduced pressure. The residue was purified by silica gel column chromatography (petroleum ether/ethyl acetate=1/1, v/v) to obtain the title product (16 g). MS(ESI+):264.0(M+H).

b) (2-Amino-5-(methoxymethyl)phenyl)dimethyl phosphine oxide

To a stirred solution of 2-iodo-4-(methoxymethyl)aniline (16g, 60.82mmol, 1.00eq.), potassium phosphate (14.20g), palladium acetate (0.68g), and 4,5-bis(diphenylphosphino)-9,9-dimethyloxanthene (1.76g) in N,N-dimethylformamide (224mL) under a nitrogen atmosphere was added dimethyl phosphine oxide (5.22g), and the reaction was stirred at 120°C for 2 hours. The mixture was cooled to room temperature. The resulting mixture was filtered and the filter cake was washed with N, N-dimethylformamide (3x5mL). The filtrate was concentrated under reduced pressure and purified by silica gel column chromatography (dichloromethane/methanol =20/1, v/v) to give the title product (12.9 g). MS($ESI_+$):214.1(M+H).

c) (2-((2,5-Dichloropyrimidin-4-yl)amino)-5-(methoxymethyl)phenyl)dimethyl phosphine oxide

(2-Amino-5-(methoxymethyl)phenyl)dimethyl phosphine oxide (1.10g), 2,4,5-trichloropyrimidine (1.23g) and N,N-diisopropylethylamine (2.00g) were added to N,N-dimethylformamide (22mL) at room temperature and stirred for 3 h. The resulting mixture was diluted with dichloromethane (30mL). The reaction was quenched by adding water (10 mL) at 0°C. The resulting mixture was extracted with dichloromethane (3x50mL). The combined organic layers were washed with saturated sodium chloride (1×50mL) and dried over anhydrous sodium sulfate. After filtration, the filtrate was concentrated under reduced pressure. Purification of the residue by silica gel column chromatography (dichloromethane/methanol=20/1, v/v) gave the title product (1.28g). MS($ESI_+$):360.0(M+H).

d) (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide

(2-((2,5-Dichloropyrimidin-4-yl)amino)-5-(methoxymethyl)phenyl)dimethylphosphine oxide (50.00mg) and (S)-7-(pyrrolidin-1-yl)-6,7,8,9-tetrahydro-5H-benzo[7]annulen-2-amine (31.98mg) were added to isopropanol (2mL), followed by hydrogen chloride in 1,4-dioxane (10 drops, 4M) and microwave radiation was applied at 130°C for 3.5h. The mixture was then cooled to room temperature and concentrated under reduced pressure. The crude product was purified by reversed-phase high performance liquid chromatography (column YMCActusTriartC18, 30*150mm, particle size 5μm, mobile phase A: water (10mmol/L ammonium bicarbonate), mobile phase B: acetonitrile, flow rate: 60 mL/min, gradient: 20%B to 50%B, 8min, wavelength: 220nm, retention time: 6.83min, column temperature: 25°C), the title product (20.2mg) was obtained.

$^1$HNMR(400MHz,DMSO-$d_6$,*ppm*)

δ11.07(s,1H),9.26(s,1H),8.52(d,*J*=4.6Hz,1H),8.17(s,1H),7.53(dd,*J*=14.0,2.0Hz,1H),7.44(q,*J*=3.1 Hz,2H),7.26(dd,*J*=8.1,2.3Hz,1H),6.97(d,*J*=8.1Hz,1H),4.42(s,2H),3.31(s,3H),3.01-2.75(m,2H),2.55(s,5H),2.50(s,2H),1.84(s,2H),1.81(s,3H),1.77(s,3H),1.70(q,*J*=3.6,3.2Hz,4H),1.5 4(s,2H).MS($ESI_+$):554.2(M+H).

**Example 2 Activity Assay**

Related compounds prepared in Example 1 were tested for enzyme activity, cell activity, and activity in vivo.

The specific structure of the positive drug 1 (BGB324) used in the activity test is as follows:

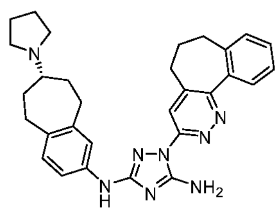

The specific structure of positive drug 2 (TP0903) is as follows:

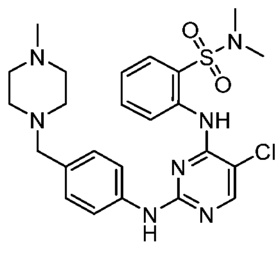

The above compounds were purchased from Shanghai Shenghong Biotechnology Co., Ltd.

**(1) AXL kinase inhibitory activity**

1. Experimental process

a) AXL enzyme (Carna, 08-107) configuration and addition: 33.33ng/μL of AXL enzyme was diluted to 0.027ng/μL (1.67×, final conc.=0.016ng/uL) with 1× enzyme buffer (1 ml of 1× enzyme buffer configured with 200μL of Enzymatic buffer kinase 5X, 10μL of 500mM $MgCl_2$, 10μL of 100mM DTT, and 6.26μL of 2500nM SEB, with the addition of 773.75μL of $H_2O$), using a BioTek (MultiFloFX) automated dispenser, compound wells and positive control wells were each spiked with 6μL of 1.67 times the final concentration of enzyme solution; 6μL of 1× Enzymatic buffer was added to the negative control wells.

b) Compound preparation and addition: the compounds prepared in Example 1 and the positive drug were diluted from 10mM to 100μM using DMSO and titrated with a compound titrator (Tecan, D300e), which automatically sprayed the desired concentration into each well, with a 1st concentration of 1μM and 1/2 log gradient dilution, for a total of 8 concentrations. Centrifugation was performed at 2500rpm for 30s, and incubation was performed at room temperature 15 minutes.

c) ATP, substrate preparation and addition: ATP (Sigma, A7699) was diluted using 1× enzyme buffer, from 10mM to 75μM (5×), resulting in a final concentration of 15μM; The substrate TKSubstrate 3-biotin (Cisbio, 61TK0BLC) was diluted with 1× enzyme buffer, from 500μM to 5μM (5×), resulting in a final concentration of 1μM; ATP was mixed with the substrate in equal volumes. Using the BioTek automatic liquid dispenser, 4μL of the mixture was added to each well; The plate was centrifuged at 2500 rpm for 30 seconds, followed by a 45-minute reaction at 25°C.

d) Assay preparation and addition: Streptavidin-XL665 (Cisbio, 610SAXLG) was diluted with HTRFK in EASE detection buffer (cisbio) from 16.67μM to 250nM (4×) with a final concentration of 62.5nM; TK Antibody- Cryptate (Cisbio) was diluted from 100× to 5× with HTRF KinEASE detection buffer (cisbio), and the final concentration was 1×; XL665 was mixed in equal volume with Antibody, and 10μL was added to each well with a BioTek automated dispenser, centrifugation was performed at 2500rpm for 30s, and the reaction was carried out at 25°C for 1 hour. At the end of the reaction, the reaction was detected by a multifunctional plate reader HTRF.

2.Data analysis

The $IC_{50}$ values of compounds for AXL kinase inhibition were obtained by fitting the dose-response curves using GraphPad Prism 5 software log(inhibitor) vs. response-Variable slope.

The inhibition rate calculation formula is as follows:

$$\text{Inhibition rate\%} = \frac{\text{Conversion readings for wells without compounds inhibition} - \text{conversion readings for samples}}{\text{Conversion readings for wells without compounds inhibition} - \text{conversion readings for wells without enzyme activity}} \times 100\%$$

3. The experimental results are detailed in the following table

Table 6: $IC_{50}$ data for AXL inhibitory activity of Example 1 compounds

| Compound | AXL inhibitory activity $IC_{50}$ (nM) |
|---|---|
| Example 1 | 4.89 |
| Positive drug 1 (BGB324) | 2 .25 |
| Positive drug 2 (TP0903) | 16.39 |

**(2) Detection of cell proliferation inhibition by compounds**

1. Experimental process

MV-4-11 cells (human myelomonocytic leukemia cell line, medium: IMDM+10% fetal bovine serum) were purchased from Nanjing Kobai Biotechnology Co. Ltd. and were cultured in an incubator at 37°C, 5% $CO_2$. The cells in logarithmic growth phase were spread in 96-well plates at a cell density of 8000 cells/well, 6000 cells/well, 5000 cells/well, 4000 cells/well and 3000 cells/well, respectively, and a blank control group was set up at the same time.

The compounds to be tested as well as the positive drug were dissolved in dimethyl sulfoxide to prepare a

10mM reservoir solution, which was stored at -80°C in a refrigerator for a long period of time. After 24 hours of cell spreading, the 10mM compound reservoir solution was diluted with dimethyl sulfoxide to obtain a 200-fold concentration of working solution (ranging from 200 to 2000μM, with a 3-fold gradient, and a total of 10 concentrations). 3μL of each concentration was added to 197μL of complete medium to further dilute it to a 3-fold concentration of working solution. Subsequently, 50μL of this working solution was added to 100μL of cell culture medium (with a final dimethyl sulfoxide concentration of 0.5%, v/v), with two replicate wells per concentration. After 72 hours of dosing treatment, 50μL of Cell Titer-Glo® (purchased from Promega) was added to each well. Fluorescence signals were measured on an Envision plate reader (PerkinElmer) following the procedure in the instruction manual, and the $IC_{50}$ value of the compound on cell proliferation inhibition was obtained by fitting the dose-response curve using the GraphPad Prism 5 software log(inhibitor) vs. response-variable slope.

Inhibition rate calculation formula:

$$\text{Inhibition rate\%} = \frac{1-（\text{Subject signal value - Blank group signal value}）}{\text{Negative control group signal value - Blank group signal value}} \times 100\%$$

Wherein:

Subject signal value: mean fluorescence signal value of cells + culture medium + compound group.
Blank group signal value: mean fluorescence signal value of culture medium group (containing 0.5% DMSO).
Negative control group signal value: mean fluorescence signal of cell + culture medium group (containing 0.5% DMSO).

2. Experimental results

The $IC_{50}$ (MV4-11, nM) of the anti-proliferative activity of the compound of Example 1 on MV4-11 cells is 6.97.

**(3) In** vivo **efficacy of MV4-11 of the compound**

inhibitory effects of test compounds as well as positive drugs on in vivo tumor growth in a transplanted tumor model of human acute monocytic leukemia cells MV-4-11 in nude mice.

1. Construction of mouse model

The logarithmic growth phase MV-4-11 cells were harvested, the cells were counted and resuspended, and then the cell concentration was adjusted to $7.0\times10^7$ cells/mL; the MV-4-11 cells were injected subcutaneously into the anterior right axilla of nude mice, and each animal was inoculated with 200μL ($14\times10^6$ cells/animal), to establish the MV-4-11 transplantation tumor model. When the tumor volume reached 100-300$mm^3$, the tumor-bearing mice with good health condition and similar tumor volume were selected.

2. Configuration of compounds

The compound as well as the positive drug, were vortexed and shaken with an appropriate solvent and then sonicated to dissolve completely and then the appropriate volume of citrate buffer was slowly added and vortexed and shaken to mix the liquids well to obtain the administered formulations at concentrations of 0.1, 0.5, and 1$mg \cdot mL^{-1}$.

Solvent control group: PEG400&citric acid buffer (20:80, v:v).

3. Animal grouping and drug administration

The modeled mice were randomly divided into groups (n=6). On the day of grouping, the relevant compounds and positive drugs were administered. The experiment was concluded after 21 days or when the tumor volume reached 2000$mm^3$ in the solvent control group (whichever occurred first). The administration volume was 10mL/kg. Both the compounds and the positive drugs were administered intragastrically once a day. Throughout the experiment, tumor dimensions and animal weights were measured twice a week to calculate tumor volume.

4. Data analysis

Tumor volume (TV) was calculated as: tumor volume ($mm^3$)=$l \times w^2/2$, wherein, l represents the long diameter of the tumor (mm); w represents the short diameter of the tumor (mm).

Relative Tumor Volume (RTV) was calculated as: RTV= $TV_t/TV_{initial}$, wherein, $TV_{initial}$ is the tumor volume measured during group administration; $TV_t$ is the tumor volume measured at each time during the administration period.

The tumor growth inhibition rate TGI (%) was calculated as: TGI=100%$\times$[1 - ($TV_{t(T)}$ - $TV_{initial(T)}$)/($TV_{t(C)}$ - $TV_{initial(C)}$)], wherein, $TV_{t(T)}$ represents the tumor volume per measurement in the treatment group; $TV_{initial(T)}$ represents the tumor volume in the treatment group at the time of group administration; $TV_{t(C)}$ represents the tumor volume per measurement in the solvent control group; and $TV_{initial(C)}$ represents the tumor volume in the solvent control group at the time of group administration.

The relative tumor proliferation rate (%T/C) was calculated as: %T/C=100%$\times$($RTV_T/RTV_C$), wherein, $RTV_T$ represents the RTV of the treatment group; $RTV_C$ represents the RTV of the solvent control group.

The experimental data were calculated and related statistical were processed using Microsoft Office Excel 2007 software.

5. The experimental results are as follows

Table 7: In vivo efficacy of compounds of Example 1

| Compound | intragastrical administration dosage (mg/kg/d) | Tumor growth inhibition rate (TGI%) | Relative tumor proliferation rate (%T/C) |
|---|---|---|---|
| Example 1 | 5 | 85 | 23 |
| Positive drug 1 (BGB324) | 20 | 32 | 71 |
| Positive drug 2 (TP0903) | 5 | 62 | 42 |
| Note: The experimental data in the table are the relevant data obtained when the experiment ends (the end of the experiment is defined as: 21 days later or when the tumor volume in the solvent control group reaches 2000$mm^3$ (whichever reaches the indicator first)). | | | |

(4) Pharmacokinetic study of compounds in ICR mice

1. Gavage prescription configuration of compounds

Each compound was prepared into a 10mg/mL stock solution with DMSO.

Mixed solvent preparation: Tween 80: PEG 400: Water=1:9:90 (v/v/v).

Accurately aspirated 450$\mu$L of DMSO stock solution of the compounds at a concentration of 10 mg/mL to a glass vial, respectively, added an appropriate volume of DMSO and mixed solvent, the ratio of solvent in the final preparation is DMSO: mixed solvent (v/v)=10:90, vortexed (or sonicated), dispersed homogeneously, to obtain the concentration of 1mg/mL of 4.5mL of the administered test solution respectively.

2. Test plan

Male 6-10 weeks old ICR mice (mouse source: Viton Lever Laboratory Animal Technology Co., Ltd.) were taken, 6 mice in each group, and the mice were fasted overnight and fed 4 hours after drug administration. On the day of the experiment, mice were given 10mg·$kg^{-1}$ of compound test solution by gavage respectively. After the administration of the drug, mice were blood sampled at 0, 5min, 15min, 30min, 1h, 2h, 4h, 8h, and 24h from the orbital region of about 100$\mu$L, which was placed in EDTA-$K_2$ anticoagulant tubes. The whole blood samples were centrifuged at 1500-1600g for 10min, and the plasma obtained from the separation was stored in a refrigerator at - 40~-20°C and used for the analysis of biological samples. Plasma concentration was determined by LC-MS/MS method.

3. Data analysis and results

Pharmacokinetic parameters were calculated using the non-compartment model in Pharsight Phoenix 7.0, and the results are detailed in the following table.

Table 8: Mouse pharmacokinetic results of the compound of Example 1

| Compound | Cmax(ng/mL) | Tmax(h) | $AUC_{0-24}$(ng.h/mL) | $T_{1/2}$(h) |
|---|---|---|---|---|
| Example 1 | 324 | 2.17 | 1300 | 1.35 |
| Positive drug 2 (TP-0903) | 26.8 | 0.25 | 52.2 | 1.20 |

**Example 3 Preparation of crystal form B of mono-p-toluenesulfonate monohydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide (compound of formula II)**

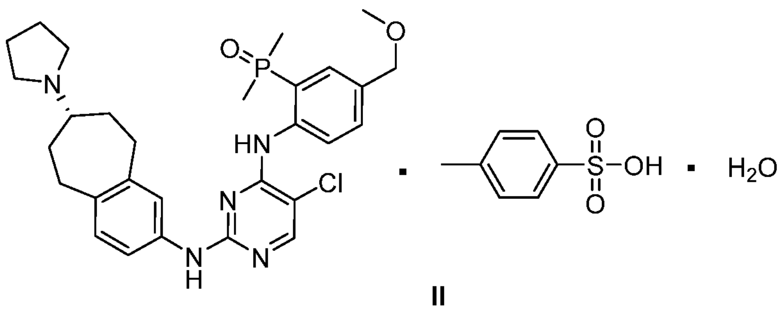

II

P-toluenesulfonic acid monohydrate (6.87g) and 99% isopropanol-water (volume percentage, 140mL) were added to a 500-mL double glass jacketed reactor and the mixture was heated to 70ºC and stirred mechanically to dissolve. (S)-(2-((5-chloro-2-((7-(pyrrolidin-1-yl)-6,7,8,9-tetrahydro-5H-benzo[7]annulen-2-yl)amino))pyrimidin-4-yl)amino)-5-(methoxymethyl)phenyl)dimethyl phosphine oxide (compound of formula IV, prepared according to the method of Example 1) (20 g) was dissolved in 99% isopropanol-water (volume percentage, 60 mL) and added dropwise to the reactor for about 15 minutes. After stirring for a few minutes, a large amount of solid was precipitated in the reactor and stirring was continued for about 15 minutes. The system was cooled to 60ºC, and methyl tert-butyl ether (200mL) was added to the reactor. After the addition, the system was stirred and ripened at 60ºC for 1 hour. After ripening , the system was cooled to 20ºC, and the stirring and ripening continued for another hour. After ripening, the system was filtered and the wet cake was dried under vacuum at 40ºC for 15 hours to obtain light yellow solid powdery crystal form B of mono-p-toluenesulfonate monohydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide: 23.67g (yield: 88.3%).

$^1$HNMR(400MHz,DMSO-d6):11.11(s,1H);9.40(br,1H);9.37(s,1H);8.56-8.54(m,1H);8.19(s,1H);7.56(dd,J=14,2Hz,1H);7.50-7.45(m,4H); 7.37(dd,J=8.2,2.2Hz, 1H);7.11(d,J=7.9,2H);7.04(d,J=8.2, 1H); 4.44(s,2H);3.48(s,3H);3.33(s,3H);3.15(s,2H);2.82-2.62(m,4H);

2.32(s,2H);2.29(s,3H);1.98(s,2H);1.85-1.83(m,2H) ;1.81(d,J=2.4Hz,3H);1.77(d,J=2.4Hz,3H);1.39(q,J=11.9Hz,2H) .

The X-ray powder diffraction pattern data is shown in Table 9 and the spectrum is shown in Figure 1; the DSC spectrum shows endothermic peaks at 90°C and 200°C, see Figure 2 for details; the TGA spectrum shows a weight loss of 2.4% at 25-150°C, see Figure 3 for specific data; see Figure 4 for detailed FT-IR spectrum, Figure 5 for detailed FT-Raman spectrum, and Figure 6 for detailed DVS spectrum.

Table 9: X-ray powder diffraction pattern data of crystal form B

| 2θ(º) | Counts | $I/I_0$(%) |
|---|---|---|
| 6.0 | 1772 | 100.0 |
| 6.3 | 1424 | 80.3 |
| 7.7 | 36 | 2.0 |
| 8.1 | 26 | 1.4 |
| 9.2 | 12 | 0.7 |
| 9.7 | 16 | 0.9 |
| 10.5 | 1448 | 81.7 |
| 11.5 | 970 | 54.8 |
| 12.3 | 215 | 12.1 |
| 12.4 | 133 | 7.5 |
| 12.6 | 169 | 9.5 |
| 13.2 | 1033 | 58.3 |
| 13.6 | 53 | 3.0 |
| 14.1 | 190 | 10.7 |
| 15.2 | 804 | 45.4 |
| 15.9 | 281 | 15.9 |
| 16.7 | 323 | 18.2 |
| 17.1 | 292 | 16.5 |
| 18.0 | 974 | 54.9 |
| 18.6 | 947 | 53.4 |
| 18.7 | 449 | 25.3 |
| 19.0 | 354 | 20.0 |
| 19.4 | 764 | 43.1 |
| 19.7 | 487 | 27.5 |
| 20.6 | 50 | 2.8 |
| 21.1 | 239 | 13.5 |
| 21.8 | 1152 | 65.0 |
| 22.6 | 844 | 47.6 |
| 23.4 | 106 | 6.0 |
| 23.7 | 232 | 13.1 |
| 24.1 | 184 | 10.4 |
| 24.4 | 181 | 10.2 |
| 24.7 | 274 | 15.5 |
| 25.1 | 227 | 12.8 |
| 25.6 | 71 | 4.0 |
| 26.3 | 177 | 10.0 |
| 26.6 | 223 | 12.6 |
| 27.0 | 371 | 20.9 |

(continued)

| 2θ(º) | Counts | $I/I_0$(%) |
|---|---|---|
| 27.7 | 249 | 14.1 |
| 28.0 | 143 | 8.1 |
| 28.3 | 226 | 12.8 |
| 28.7 | 109 | 6.2 |
| 28.8 | 303 | 17.1 |
| 29.4 | 459 | 25.9 |
| 30.2 | 281 | 15.8 |
| 32.0 | 81 | 4.5 |
| 33.1 | 68 | 3.9 |
| 33.9 | 152 | 8.6 |
| 35.1 | 58 | 3.3 |
| 35.7 | 44 | 2.5 |

**Example 4 Preparation of crystal form B of mono-p-toluenesulfonate monohydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide (compound of formula II)**

(S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide (compound of formula IV, 2.34 g), p-toluenesulfonic acid monohydrate (0.80 g) and ethanol (11.7 mL) were added to a 50-mL glass bottle and the mixture was dissolved with magnetic stirring at room temperature. A large amount of solid was precipitated after about 10 minutes. Continued stirring for about 30 minutes. Then, isopropyl acetate (11.7mL) was added to the glass bottle. After addition, the mixture was stirred and ripened for about 30 minutes at room temperature. After ripening, isopropyl acetate (11.7 mL) was added to the glass bottle and continued ripening for about 1.5 hours. After ripening, filtration was carried out and the wet filter cake was dried under vacuum at 40ºC for 24 hours to obtain light yellow solid powdery crystal form B of mono-p-toluenesulfonate monohydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide: 2.35g (yield: 74.8%).

**Example 5 Preparation of single crystal of crystal form B of mono-p-toluenesulfonate monohydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide (compound of formula II)**

Single crystal culture method: the sample of monohydrate crystal form B was dissolved in 95% ethanol-water, prepared as a suspension of 9.1 mg/mL, warmed up to 50-60ºC to dissolve, filtered and precipitated by cooling down at room temperature that is to obtain the elongated needle-like single crystals, and its crystallographic parameters and structural data table are as follows.

Table 10: crystal parameters and structural data of crystal form B

| Analytical data | |
|---|---|
| Molecular formula | $C_{29}H_{38}ClN_5O_2P{\cdot}C_7H_7O_3S{\cdot}H_2O$ |
| Molecular weight | 744.28 |
| Crystal system | Orthorhombic |
| Space group | $P2_12_12_1$ |

(continued)

| Analytical data | |
|---|---|
| Cell parameters | a= 7.9891 (3) Å, α=90° |
| | b= 15.7648(7)Å, β=90° |
| | c= 29.3381 (14)Å, γ=90° |
| Crystal axis ratio | a/b=0.5068, b/c=0.5367, c/a=3.6723 |
| Z | 4 |
| Unit cell volume | 3695.0(3)$Å^3$ |
| Theoretical density | 1.338$Mg/m^3$ |
| $R_1$ | 0.050 |
| $WR_2$ | 0.114 |
| GOOF=S | 1.03 |
| $R_{(int)}$ | 0.082 |
| Flackparameter | 0.019(19) |

According to the analysis of X-ray single crystal diffraction data, crystal form B is a monohydrate.

**Example 6 Preparation of crystal form C of mono-p-toluenesulfonate sesquihydrate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide (compound of formula III)**

The crystal form B monohydrate was dissolved in water, prepared into a suspension of about 3 mg/mL and warmed to 50-60ºC to dissolve, the solution was filtered and precipitated at room temperature to obtain the elongated needle-like single crystals, i.e., crystal form C sesquihydrate.

The XRPD pattern is shown in Figure.7 and the data are detailed in Table 11:

Table 11: X-ray powder diffraction pattern data of crystal form C

| 2θ (º) | counts | $I/I_0$ (%) |
|---|---|---|
| 6.0 | 105 | 22.2 |
| 10.8 | 60 | 12.8 |
| 12.1 | 227 | 48.2 |
| 13.4 | 322 | 68.5 |
| 15.0 | 220 | 46.7 |
| 16.9 | 221 | 46.9 |
| 18.4 | 471 | 100.0 |
| 19.0 | 176 | 37.5 |
| 19.3 | 238 | 50.5 |
| 19.8 | 306 | 65.0 |
| 20.9 | 154 | 32.8 |
| 21.8 | 310 | 65.9 |
| 23.2 | 172 | 36.6 |
| 23.6 | 231 | 49.1 |
| 24.3 | 213 | 45.2 |

(continued)

| 2θ (º) | counts | $I/I_0$ (%) |
|---|---|---|
| 25.5 | 62 | 13.1 |
| 26.6 | 103 | 21.8 |
| 27.7 | 38 | 8.0 |
| 29.7 | 93 | 19.7 |

The crystallographic parameters and structural data of crystal form C single crystal are detailed as described in Table 12;

Table 12: crystallographic parameters and structural data of crystal form C sesquihydrate

| Analytical data | |
|---|---|
| Molecular formula | $2(C_7H_7O_3S)\cdot 2(C_{29}H_{38}ClN_5O_2P)\cdot 3(H_2O)$ |
| Molecular weight | 1506.54 |
| Crystal system | Orthorhombic |
| Space group | $P2_12_12_1$ |
| Cell parameters | a=8.4802(8)Å, α=90° |
| | b=15.1272(14)Å, β=90° |
| | c=29.429(3)Å, γ=90° |
| Crystal axis ratio | a/b=0.5606, b/c=0.5140, c/a=3.4703 |
| Z | 2 |
| Unit cell volume | 3775.2(7)$Å^3$ |
| Theoretical density | 1.325$Mg/m^3$ |
| $R_1$ | 0.053 |
| $WR_2$ | 0.114 |
| GOOF=S | 1.06 |
| $R_{(int)}$ | 0.041 |
| Flackparameter | 0.029(12) |

From the above single crystal analysis data, it can be determined that the crystal form C is sesquihydrate.

**Example 7 Solubility related measurements**

Method of solubility test in water for crystal form B of mono-p-toluenesulfonate monohydrate of the compound of formula IV: 0.5g of crystal form B were accurately weighted, added water dropwise, recorded the amount of solvent added and the dissolution state of crystal form B. When crystal form B was completely dissolved, recorded the amount of solvent added and calculate the critical saturation solubility; if the addition of more than 50 mL of the solvent is still unable to dissolve clear, then take sample centrifugation to detect saturation solubility.

Method of solubility test for crystal form B of mono-p-toluenesulfonate monohydrate of the compound formula IV in various pH values: 0.2g of crystal form B were accurately weighted, added solvent dropwise, recorded the amount of solvent added and the dissolution state of crystal form B. When crystal form B was completely dissolved, recorded the amount of solvent added and calculate the critical saturation solubility; if the addition of more than 50 mL of solvent is still unable to dissolve clear, then take sample centrifugation to detect saturation solubility. The following table shows the saturated solubility of the compound of formula IV. The solubility of crystal form B of mono-p-toluenesulfonate monohydrate of the compound formula IV is detailed in the following table:

Table 13: solubility of crystal form B in various media

| pH value | Medium | Solubility (mg/mL) |
|---|---|---|
| pH1.0 | Dilute hydrochloric acid with water | 87.2 |
| pH2.0 | Dilute hydrochloric acid with water | 4.7 |
| pH4.5 | A mixture of sodium acetate trihydrate and glacial acetic acid is dissolved in water and diluted | 2 |
| pH6.0 | A mixture of anhydrous potassium dihydrogen phosphate and sodium hydroxide is dissolved in water and diluted | 1.9 |
| Water | A mixture of anhydrous potassium dihydrogen phosphate and sodium hydroxide is dissolved in water and diluted | 1.4 |
| pH6.8 | A mixture of anhydrous potassium dihydrogen phosphate and sodium hydroxide is dissolved in water and diluted | 2.1 |
| pH7.4 | A mixture of anhydrous potassium dihydrogen phosphate and sodium hydroxide is dissolved in water and diluted | 2.1 |

**Example 8 Preparation of crystal form A of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide (compound of formula IV)**

200mg of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide prepared according to the method of Example 1 was added to a 3mL glass bottle successively with 2mL of purified water, and the sample was stirred magnetically for 6 hours at room temperature. After 6h, the sample was centrifuged and the wet sample was dried under reduced pressure at 40ºC for 21h to obtain 176mg of crystal form A in 88.0% yield, whose XRPD pattern is detailed in Figure 8, and the data of the X-ray powder diffraction pattern is detailed in Table 14.

Table 14: X-ray powder diffraction pattern data of crystal form A

| 2θ(º) | Counts | $I/I_0$(%) |
|---|---|---|
| 4.1 | 45 | 15.4 |
| 5.6 | 42 | 14.3 |
| 7.6 | 109 | 36.9 |
| 10.2 | 107 | 36.4 |
| 10.9 | 13 | 4.4 |
| 12.6 | 59 | 20.1 |
| 13.0 | 44 | 14.8 |
| 15.2 | 18 | 6.2 |
| 17.6 | 246 | 83.4 |
| 19.7 | 85 | 28.9 |
| 20.3 | 184 | 62.5 |
| 20.9 | 294 | 100.0 |
| 22.2 | 60 | 20.5 |
| 23.2 | 22 | 7.5 |
| 24.6 | 19 | 6.6 |
| 27.0 | 40 | 13.6 |

(continued)

| 2θ(º) | Counts | $I/I_0$(%) |
|---|---|---|
| 28.8 | 14 | 4.6 |
| 37.0 | 6 | 2.1 |
| 37.7 | 12 | 4.1 |

**Example 9 Physicochemical stability study of crystal form of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide**

The free base crystal form A of the compound of formula IV and the crystal form B of mono-p-toluenesulfonate of the compound of formula IV were placed at 40ºC/75% RH, 60ºC, 75% RH, 92.5% RH and light exposure, respectively, to test the stability, the specific test method was referred to the requirements of the General Rules 9001 for the stability testing of raw pharmaceutical materials and preparations in the 2020 edition of the Chinese Pharmacopoeia. Use XRPD and HPLC to detect the physical and chemical stability of the samples. The detailed results are shown in the table below:

Table 15: summary of stability test results of crystal form B

| Placement conditions | Crystal form/purity (A%) | |
|---|---|---|
| | Before placement | After placement |
| 40ºC/75%RH, 5 days | Crystal form B<br>Purity: 99.81<br>Maximum single impurity: 0.04 | Crystal form B<br>Purity: 99.81<br>Maximum single impurity: 0.04 |
| 40ºC/75%RH, 10 days | | Crystal form B<br>Purity: 99.82<br>Maximum single impurity: 0.04 |
| 40ºC/75%RH, 30 days | | Crystal form B<br>Purity: 99.81<br>Maximum single impurity: 0.04 |
| 60ºC, 5 days | | Crystal form B<br>Purity: 99.81<br>Maximum single impurity: 0.04 |
| 60ºC, 10 days | | Crystal form B<br>Purity: 99.82<br>Maximum single impurity: 0.04 |
| 60ºC, 30 days | | Crystal form B<br>Purity: 99.80<br>Maximum single impurity: 0.04 |
| 75%RH, 5 days | | Crystal form B<br>Purity: 99.81<br>Maximum single impurity: 0.04 |
| 75%RH, 10 days | | Crystal form B<br>Purity: 99.82 |

(continued)

| Placement conditions | Crystal form/purity (A%) | |
|---|---|---|
| | Before placement | After placement |
| | | Maximum single impurity: 0.04 |
| 75%RH, 30 days | | Crystal form B<br>Purity: 99.80<br>Maximum single impurity: 0.04 |
| 92.5%RH, 5 days | | Crystal form B<br>Purity: 99.81<br>Maximum single impurity: 0.04 |
| 92.5%RH, 10 days | | Crystal form B<br>Purity: 99.82<br>Maximum single impurity: 0.04 |
| Light, 5 days | | Crystal form B<br>Purity: 99.67<br>Maximum single impurity: 0.05 |
| Light, 10 days | | Crystal form B<br>Purity: 99.60<br>Maximum single impurity: 0.10 |

Table 16: stability data of free base crystal form A

| Placement conditions | Crystal form/purity (A%) | |
|---|---|---|
| | Before placement | After placement |
| 60ºC, 5 days | Crystal form A<br>Purity: 99.33<br>Maximum single impurity: 0.05 | Crystal form A<br>Purity: 96.39<br>Maximum single impurity: 0.75 |
| 60ºC, 10 days | | Crystal form A<br>Purity: 94.49<br>Maximum single impurity: 1.21 |
| 60ºC, 30 days | | Not detected<br>Purity: 89.65<br>Maximum single impurity: 2.02 |

**Example 10 Preparation of salts form and crystal form of other acid of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide (compound of formula IV )**

**and crystal form thereof**

About 50 mg of the compound of formula IV (prepared according to the method of Example 1) and 1.05 equivalents of acid (hydrochloric acid, with the molar ratio of acid to compound IV set at 2.10) were taken separately, added 1mL of solvent to the mixture and stirred at room temperature for 2 days. The resulting clarified solution was attempted to crystallize by stirring and slow evaporation at 5ºC, and the solid was separated by centrifugation and dried at 40 ºC under blast or reduced pressure for 2-5 hours before being used for XRPD characterization.

Table 17: salification results of compound of formula IV

| Acid | Salification solvent | Result | |
|---|---|---|---|
| | | Salification pattern | Salification results (molar ratio of compound of formula IV to acid radical) |
| Methane sulfonic acid | Toluene<br>Ethanol/n-Heptane | Crystal form | 1: 1 |
| Hydrochloric acid | Ethanol/n-Heptane<br>Isopropyl alcohol/n-heptane<br>Acetone/n-Heptane<br>Ethyl acetate/n-heptane | Crystal form | 1: 1 |
| Hydrochloric acid | Acetone | Crystal form | 1: 2 |
| Phosphoric acid | Isopropyl alcohol/n-heptane<br>Acetone/n-heptane<br>Ethyl acetate/n-heptane | Not finalized | 1: 1 |
| Hippuric acid | Acetone/n-heptane | Crystal form | 1: 1 |
| Sulfuric acid | Acetone/n-heptane<br>Ethyl acetate/n-heptane | Not finalized | 1: 1 |
| Hydrobromic acid | Acetone/n-heptane<br>Ethyl acetate/n-heptane | Crystal form | 1: 1 |
| Benzenesulfonic | Acetone/n-heptane | Crystal form | 1: 1 |
| acid | | | |
| Oxalic acid | Ethanol/n-Heptane<br>Isopropyl alcohol/n-Heptane<br>Acetone/n-Heptane | Crystal form | 1: 1 |
| Fumaric acid | Acetone/n-Heptane | Crystal form | 1: 1 |
| Citric acid | Acetone/n-Heptane | Crystal form | 1: 1 |
| Succinic acid | Ethanol/n-Heptane<br>Isopropyl alcohol/n-Heptane<br>Acetone/n-Heptane<br>Ethyl acetate/n-Heptane<br>Acetonitrile-Water/n-Heptane | No salt is produced | |

(continued)

| Acid | Salification solvent | Result | |
|---|---|---|---|
| | | Salification pattern | Salification results (molar ratio of compound of formula IV to acid radical) |
| Maleic acid | Ethanol/n-Heptane<br>Isopropyl alcohol/n-Heptane<br>Acetone/n-Heptane<br>Ethyl acetate/n-Heptane<br>Acetonitrile-Water/n-Heptane | No salt is produced | |
| Adipic acid | Ethanol/n-Heptane<br>Isopropyl alcohol/n-Heptane<br>Acetone/n-Heptane<br>Ethyl acetate/n-Heptane<br>Acetonitrile-Water/n-Heptane | No salt is produced | |
| L-(+)-tartaric acid | Ethanol/n-Heptane<br>Isopropyl alcohol/n-Heptane<br>Acetone/n-Heptane<br>Ethyl acetate/n-Heptane<br>Acetonitrile -Water/n-Heptane | No salt is produced | |
| D-glucuronic acid | Ethanol/n-Heptane<br>Isopropyl alcohol/n-Heptane<br>Acetone/n-Heptane<br>Ethyl acetate/n-Heptane<br>Acetonitrile-Water/n-Heptane | No salt is produced | |
| L-ascorbic acid | Ethanol/n-Heptane<br>Isopropyl alcohol/n-Heptane<br>Acetone/n-Heptane<br>Ethyl acetate/n-Heptane<br>Acetonitrile-Water/n-Heptane | No salt is produced | |

(continued)

| Acid | Salification solvent | Result | |
|---|---|---|---|
| | | Salification pattern | Salification results (molar ratio of compound of formula IV to acid radical) |
| L-malic acid | Ethanol/n-Heptane<br>Isopropyl alcohol/n-Heptane<br>Acetone/n-Heptane<br>Ethyl acetate/n-Heptane<br>Acetonitrile-Water/n-Heptane | | No salt is produced |
| Benzoic acid | Ethanol/n-Heptane<br>Isopropyl alcohol/n-Heptane<br>Acetone/n-Heptane<br>Ethyl acetate/n-Heptane<br>Acetonitrile-Water/n-Heptane | | No salt is produced |
| Gentisic acid | Ethanol/n-Heptane<br>Isopropyl alcohol/n-Heptane<br>Acetone/n-Heptane<br>Ethyl acetate/n-Heptane<br>Acetonitrile-Water/n-Heptane | | No salt is produced |
| L-glutamic acid | Ethanol/n-Heptane<br>Isopropyl alcohol/n-Heptane<br>Acetone/n-Heptane<br>Ethyl acetate/n-Heptane<br>Acetonitrile-Water/n-Heptane | | No salt is produced |
| Acetic acid | Ethanol/n-Heptane<br>Isopropyl alcohol/n-Heptane<br>Acetone/n-Heptane<br>Ethyl acetate/n-Heptane<br>Acetonitrile-Water/n-Heptane | | No salt is produced |

(continued)

| Acid | Salification solvent | Result | |
|---|---|---|---|
| | | Salification pattern | Salification results (molar ratio of compound of formula IV to acid radical) |
| stearic acid | Ethanol/n-Heptane<br>Isopropyl alcohol/n-Heptane<br>Acetone/n-Heptane<br>Ethyl acetate/n-Heptane<br>Acetonitrile-Water/n-Heptane | | No salt is produced |

Specifically, the preparation method for mesylate of the compound of formula IV is as follows: About 50mg of the compound of formula IV and 1.05 equivalents of methane sulfonic acid were taken separately, 1mL of toluene were added to the mixture and stirred at room temperature for 2 days. The resulting liquid was then crystallized by slow volatilization under stirring at 5ºC. The solid was separated by centrifugation and dried under reduced pressure at 40ºC for 2-5h to obtain the mesylate crystal form of (S)-(2-((5-chloro-2-((7-(pyrrolidin-1-yl)-6,7,8,9-tetrahydro-5H-benzo[7]annulen-2-yl)amino))-pyrimidin-4-yl)amino)-5-(methoxymethyl)phenyl)dimethyl phosphine oxide with the XRPD as shown in Figure 9.

The X-ray powder diffraction (XRPD) spectrum of the hydrochloride, dihydrochloride, phosphate, hippurate, sulfate, hydrobromide, benzenesulfonate, oxalate, fumarate, and citrate of the compound of formula IV are shown in Figure 10-19, respectively.

**Example 11 Preparation of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphineoxide or hydrate thereof (compound of formula I)**

The crystal form B prepared in Example 4 was dried under reduced pressure at 40°C for 16 hours to prepare hydrate of the mono-p-toluenesulfonate of the compound of formula I (X=0~1), and the sample was taken for XRPD detection, and the X-ray powder diffraction pattern is detailed in Figure 20.

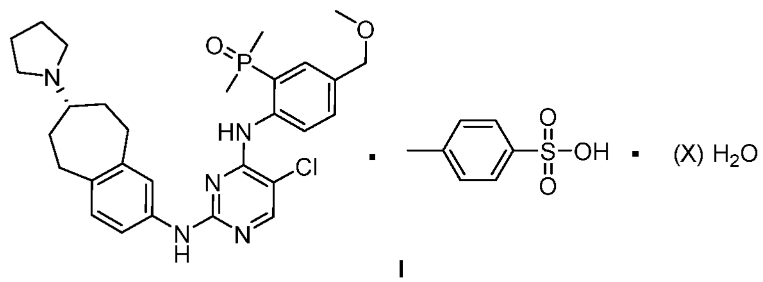

I

**Claims**

1. A mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or a hydrate thereof.

2. The mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof of claim 1, wherein the specific structure is shown in formula I:

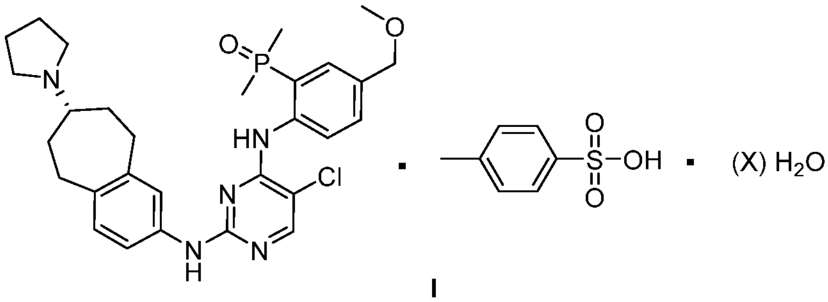

I ;

wherein X=0~2, further, X=0~1 or 1.5; further, X is 0, 0.25, 0.5, 0.7, 1, 1.25, 1.5 or 1.75.

**3.** A crystal form of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or a hydrate thereof, wherein the X-ray powder diffraction pattern has diffraction peaks at 2θ of 6.0º±0.2º, 6.3º±0.2º, 10.5º ±0.2º, 13.2º±0.2º and 21.8º ±0.2º;

further, the X-ray powder diffraction pattern has diffraction peaks at 2θ of 6.0º±0.2º, 6.3º±0.2º, 10.5º±0.2º, 11.5º±0.2º, 13.2º±0.2º, 15.2º±0.2º, 18.0º±0.2º, 18.6º±0.2º, 21.8º±0.2º and 22.6º±0.2º; further, the X-ray powder diffraction pattern has diffraction peaks at 2θ of 6.0º±0.2º, 6.3º±0.2º, 10.5º±0.2º, 11.5º±0.2º, 13.2º±0.2º, 15.2º±0.2º, 18.0º±0.2º, 18.6º±0.2º, 18.7º±0.2º, 19.4º±0.2º, 19.7°±0.2°, 21.8°±0.2°, 22.6°±0.2° and 29.4°±0.2°;

further, the X-ray powder diffraction pattern has diffraction peaks at 2θ of 6.0º±0.2º, 6.3º±0.2º, 10.5º±0.2º, 11.5º±0.2º, 12.3º±0.2º, 12.4º±0.2º, 12.6º±0.2º, 13.2º±0.2º, 14.1º±0.2º, 15.2º±0.2º, 15.9º±0.2º, 16.7º±0.2º, 17.1º±0.2º, 18.0º±0.2º, 18.6º±0.2º, 18.7º±0.2º, 19.0º±0.2º, 19.4º±0.2º, 19.7º±0.2º, 21.1º±0.2º, 21.8º±0.2º, 22.6º±0.2º, 23.3º±0.2º, 23.7º±0.2º, 24.1º±0.2º, 24.4º±0.2º, 24.7º±0.2º, 25.1º±0.2º, 26.2º±0.2º, 26.6º±0.2º, 27.0º±0.2º, 27.7º±0.2º, 28.0º±0.2º, 28.3º±0.2º, 28.7º±0.2º, 28.8º±0.2º, 29.4º±0.2º, 30.2º±0.2º and 33.9º±0.2º.

**4.** The mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof of claim 1, which is a monohydrate, and the specific structure of the monohydrate is shown in formula II:

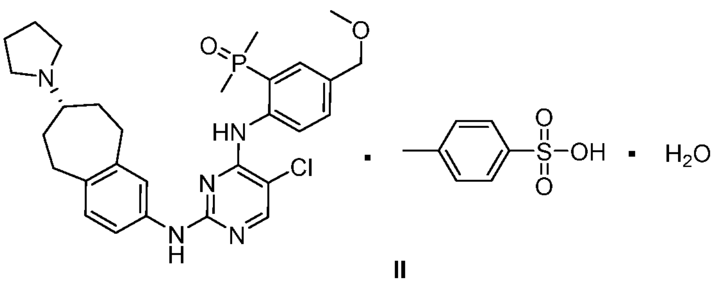

II .

**5.** The mono-p-toluenesulfonate or hydrate thereof of claim 4, wherein the X-ray powder diffraction pattern of the monohydrate crystal form has diffraction peaks at 2θ of 6.0º±0.2º, 6.3º±0.2º, 10.5º±0.2º, 13.2º±0.2º and 21.8º±0.2º;

further, the X-ray powder diffraction pattern has diffraction peaks at 2θ of 6.0º±0.2º, 6.3º±0.2º, 10.5°±0.2°, 11.5°±0.2°, 13.2°±0.2°, 15.2°±0.2°, 18.0°±0.2°, 18.6°±0.2°, 21.8°±0.2°, and 22.6°±0.2°; further, the X-ray powder diffraction pattern has diffraction peaks at 2θ of 6.0º±0.2º, 6.3º±0.2º, 10.5º±0.2º, 11.5º±0.2º, 13.2º±0.2º, 15.2º±0.2º, 18.0º±0.2º, 18.6º±0.2º, 18.7º±0.2º, 19.4º±0.2º, 19.7º±0.2º, 21.8º±0.2º, 22.6º±0.2º and 29.4º±0.2º;

further, the X-ray powder diffraction pattern has diffraction peaks at 2θ of 6.0º±0.2º, 6.3º±0.2º, 10.5º±0.2º, 11.5º±0.2º, 12.3º±0.2º, 12.4º±0.2º, 12.6º±0.2º, 13.2º±0.2º, 14.1º±0.2º, 15.2º±0.2º, 15.9º±0.2º, 16.7º±0.2º, 17.1º±0.2º, 18.0º±0.2º, 18.6º±0.2º, 18.7º±0.2º, 19.0º±0.2º, 19.4º±0.2º, 19.7º±0.2º, 21.1º±0.2º, 21.8º±0.2º, 22.6º±0.2º, 23.3º±0.2º, 23.7º±0.2º, 24.1º±0.2º, 24.4º±0.2º, 24.7º±0.2º,

25.1º±0.2º, 26.2º±0.2º, 26.6º±0.2º, 27.0º±0.2º, 27.7º±0.2º, 28.0º±0.2º, 28.3º±0.2º, 28.7°±0.2°, 28.8°±0.2°, 29.4°±0.2°, 30.2°±0.2° and 33.9°±0.2°;
further, the X-ray powder diffraction expressed in an angle of 2θ has a pattern shown in Figure 1.

**6.** The mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof of claim 1, which is a sesquihydrate, and the specific structure of the sesquihydrate is shown in formula III:

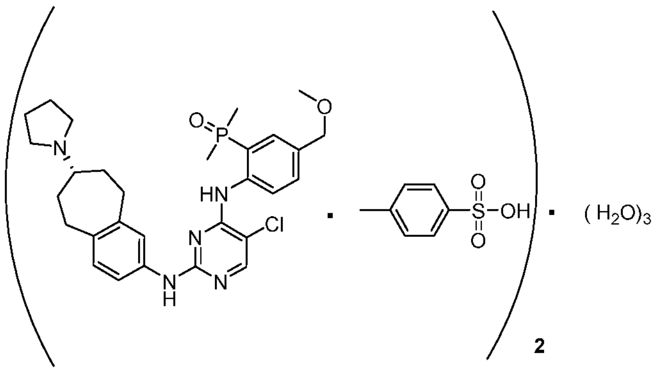

III

.

**7.** The mono-p-toluenesulfonate or hydrate thereof of claim 6, wherein the X-ray powder diffraction pattern of the sesquihydrate crystal form has diffraction peaks at 2θ of 13.4º±0.2º, 18.4°±0.2 °, 19.3°±0.2°, 19.8°±0.2° and 21.8°±0.2°;

further, the X-ray powder diffraction pattern has diffraction peaks at 2θ of 12.1º±0.2º, 13.4º±0.2º, 15.0°±0.2°, 16.9°±0.2°, 18.4°±0.2°, 19.3°±0.2°, 19.8°±0.2°, 21.8°±0.2°, 23.6°±0.2° and 24.3°±0.2°; further, the X-ray powder diffraction pattern has diffraction peaks at 2θ of 6º±0.2º, 10.8º±0.2º, 12.1º±0.2º, 13.4º±0.2º, 15.0º±0.2º, 16.9º±0.2º, 18.4º±0.2º, 19.0º±0.2º, 19.3º±0.2º, 19.8º±0.2º, 20.9º±0.2º, 21.8º±0.2º, 23.2º±0.2º, 23.6º±0.2º, 24.3º±0.2º, 25.5º±0.2º and 26.6º±0.2º;
further, the X-ray powder diffraction expressed in an angle of 2θ has a pattern shown in Figure 7.

**8.** Method for preparing the mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof of claim 1, comprising the step of salifying the compound of formula IV with p-toluenesulfonic acid, the compound of formula IV having the following structure:

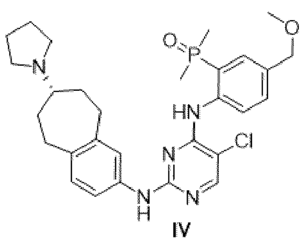

IV

.

**9.** A crystal form composition, wherein the mono-p-toluenesulfonate or hydrate thereof of any one of claims 1-7, or the mono-p-toluenesulfonate or hydrate thereof prepared according to claim 8, constitutes more than 50% by weight of the crystal form composition;

further, the mono-p-toluenesulfonate or hydrate thereof constitutes more than 80% by weight of the crystal form composition
further, the mono-p-toluenesulfonate or hydrate thereof constitutes more than 90% by weight of the crystal form composition;
further, the mono-p-toluenesulfonate or hydrate thereof constitutes more than 95% by weight of the crystal form composition.

10. A pharmaceutical composition of mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide, comprising the mono-p-toluenesulfonate or hydrate thereof of any one of claims 1-7, the mono-p-toluenesulfonate or hydrate thereof prepared according to claim 8, or the crystal form composition of claim 9.

11. Use of the mono-p-toluenesulfonate of (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide or hydrate thereof of any one of claims 1-7, the mono-p-toluenesulfonate or hydrate thereof prepared according to claim 8, the crystal form composition of claim 9, or the pharmaceutical composition of claim 10, for the preparation of medicaments used in the prevention and/or treatment of AXL kinase-mediated diseases or disease states.

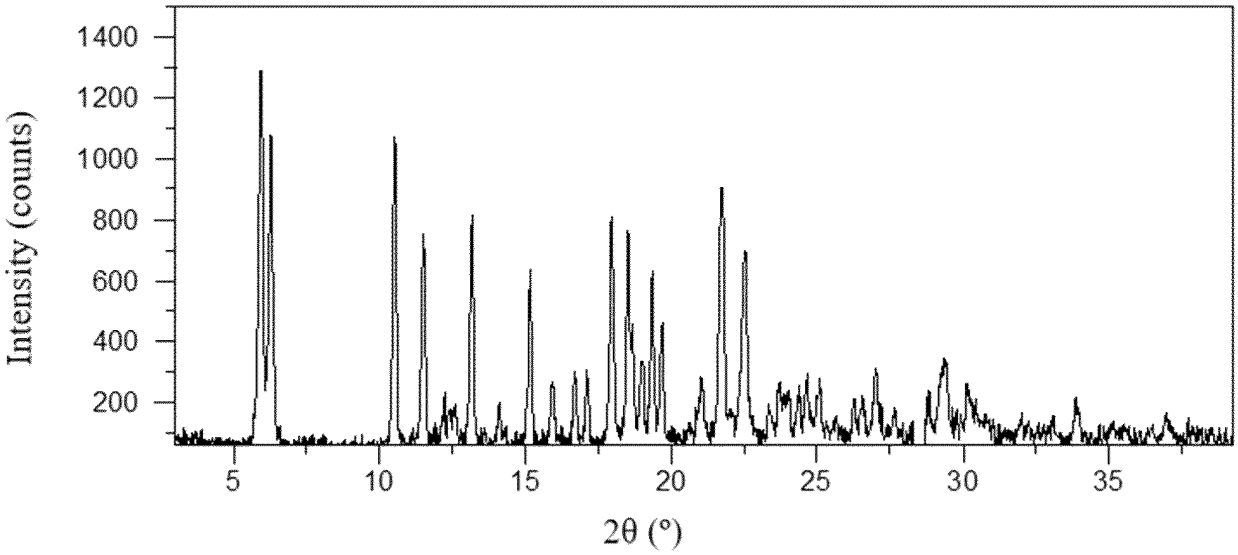


Figure.1

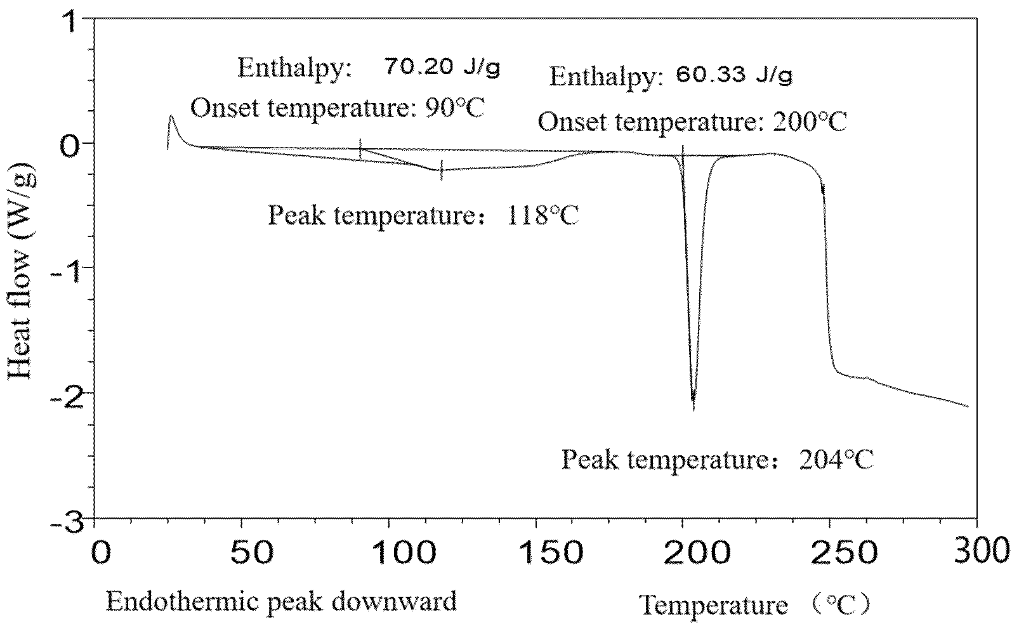


Figure.2

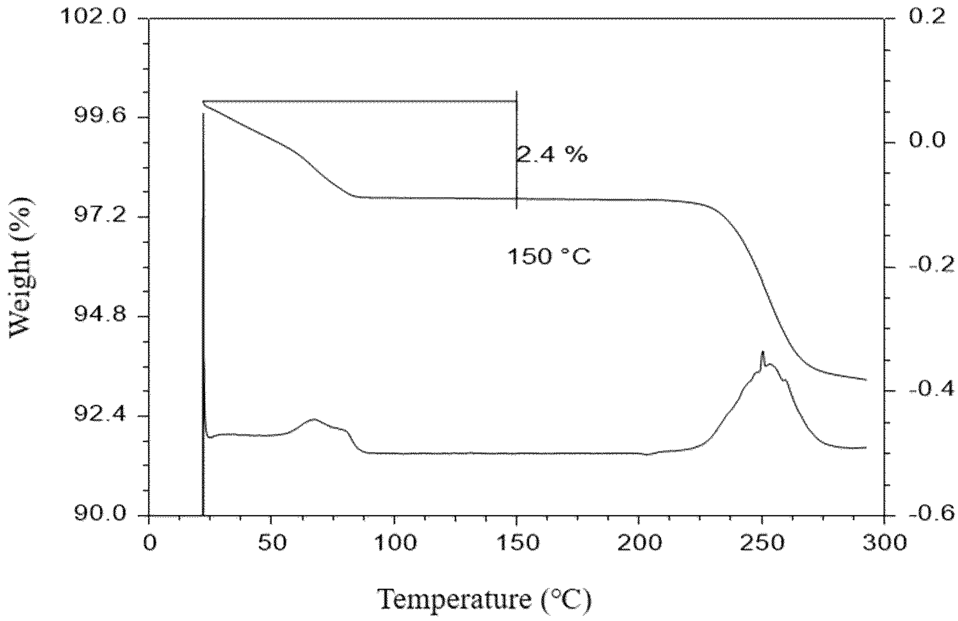


Figure.3

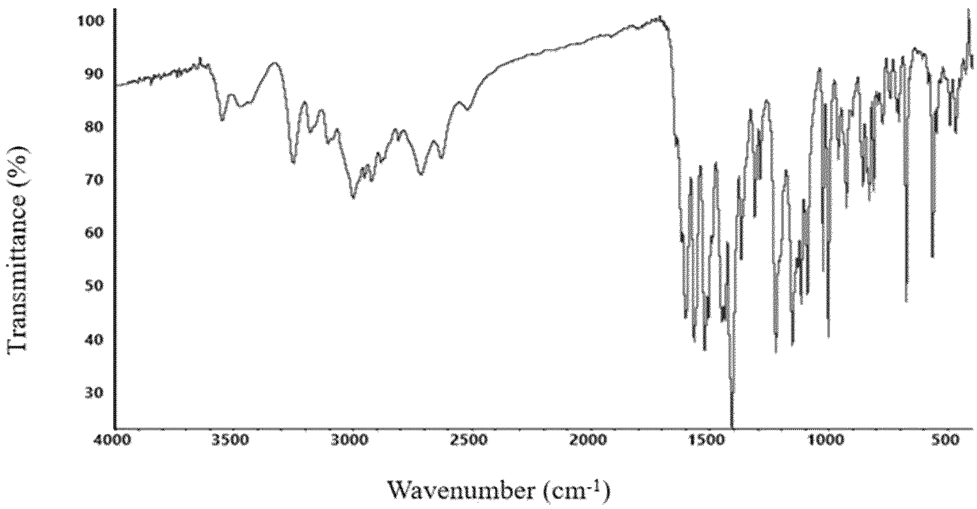


Figure.4

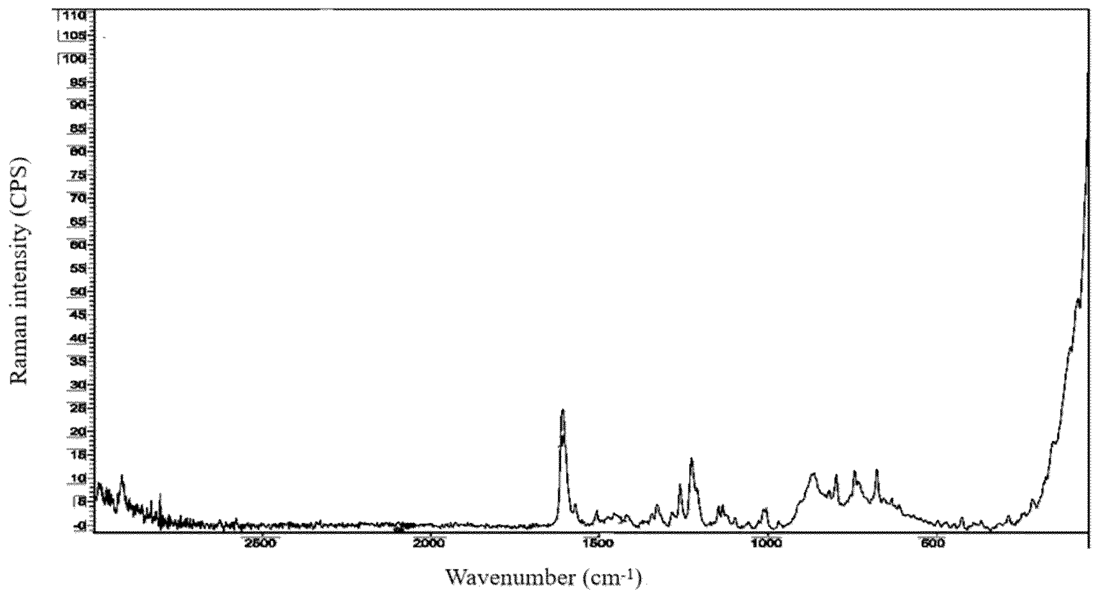


Figure.5

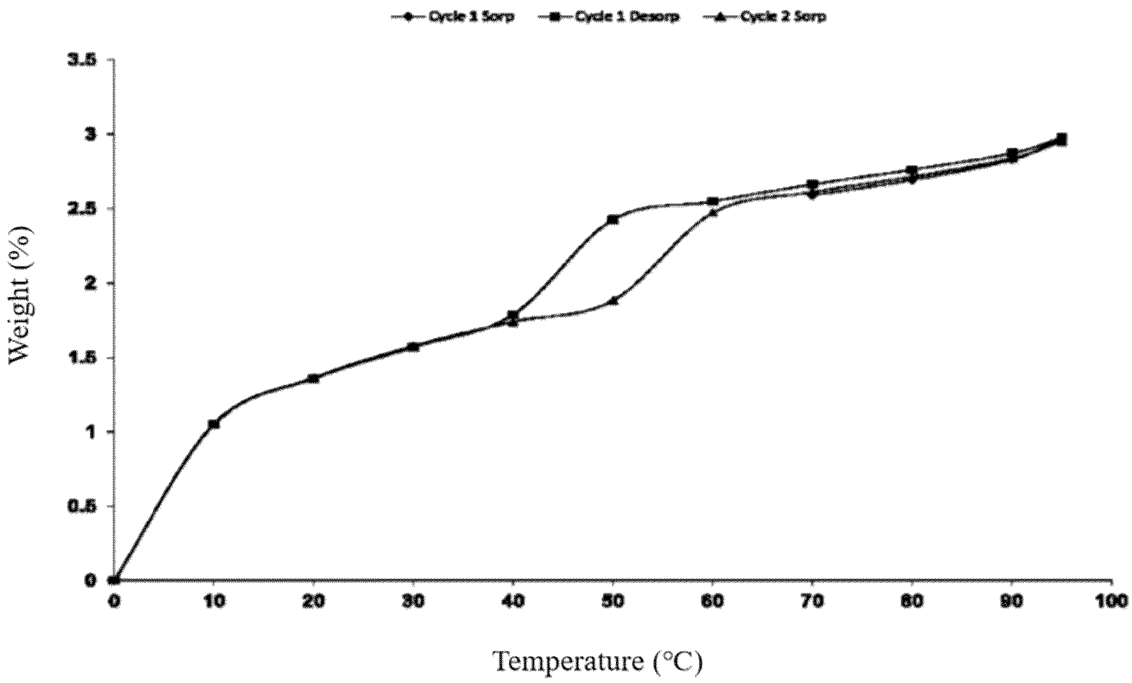


Figure.6

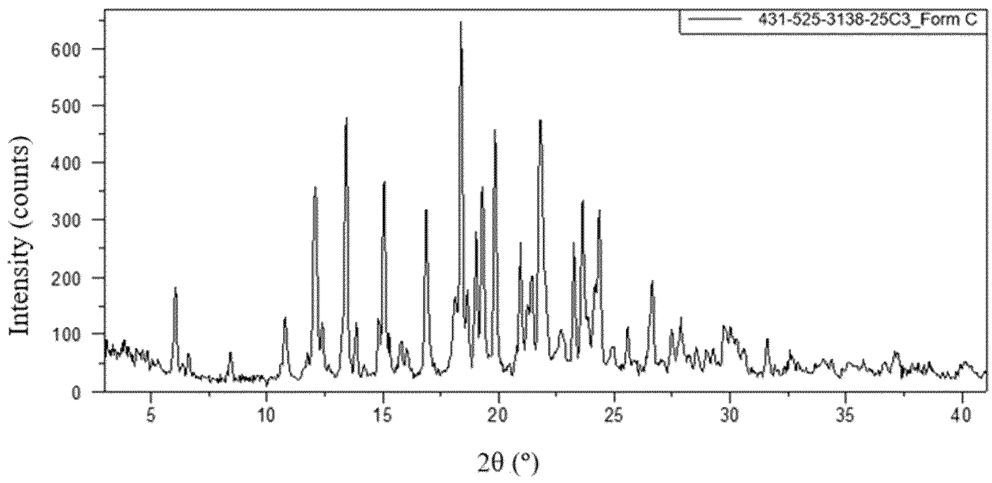


Figure.7

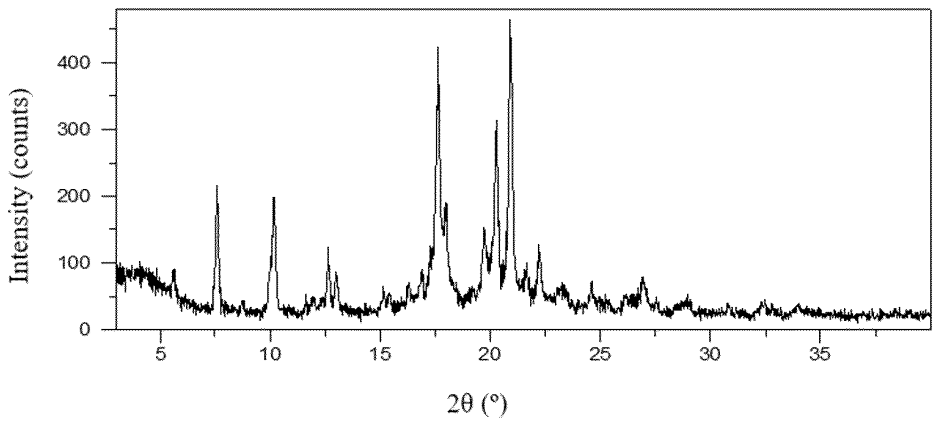


Figure.8

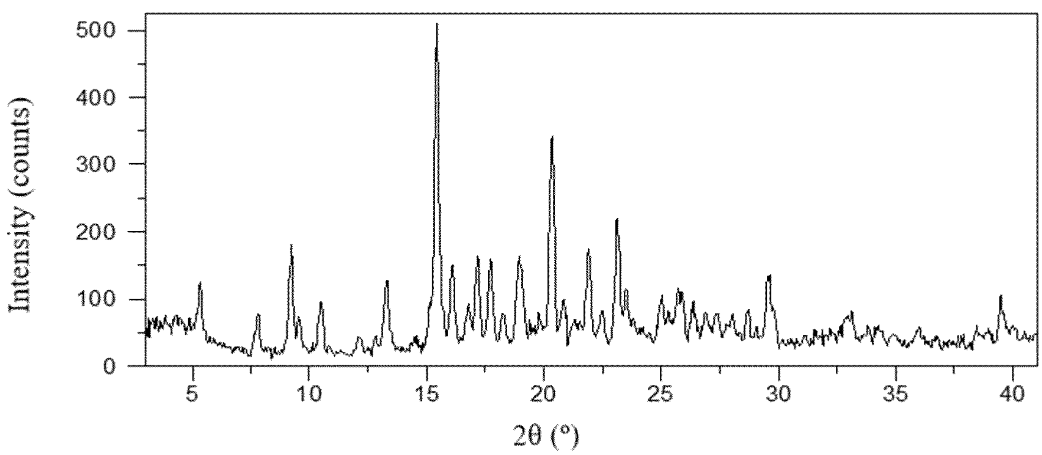


Figure.9

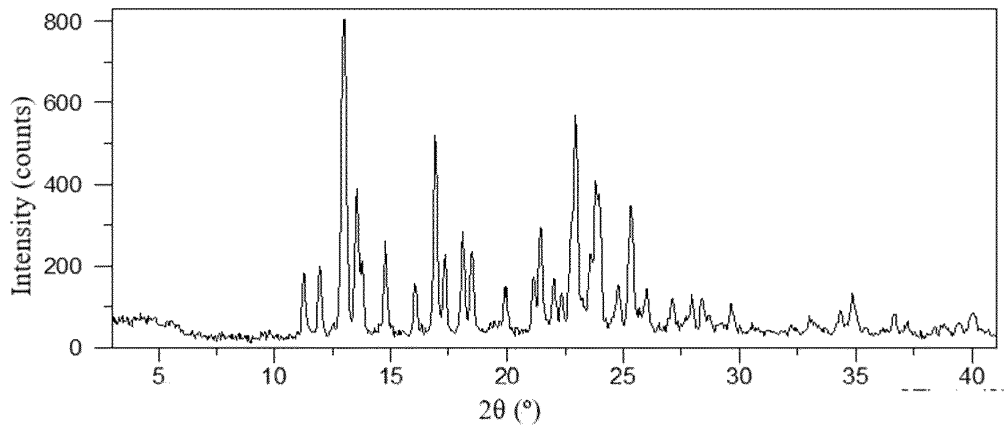


Figure.10

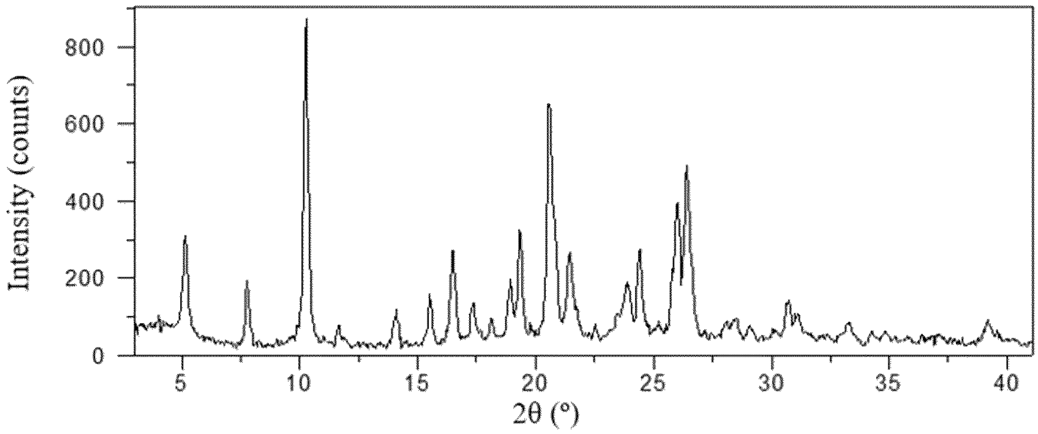


Figure.11

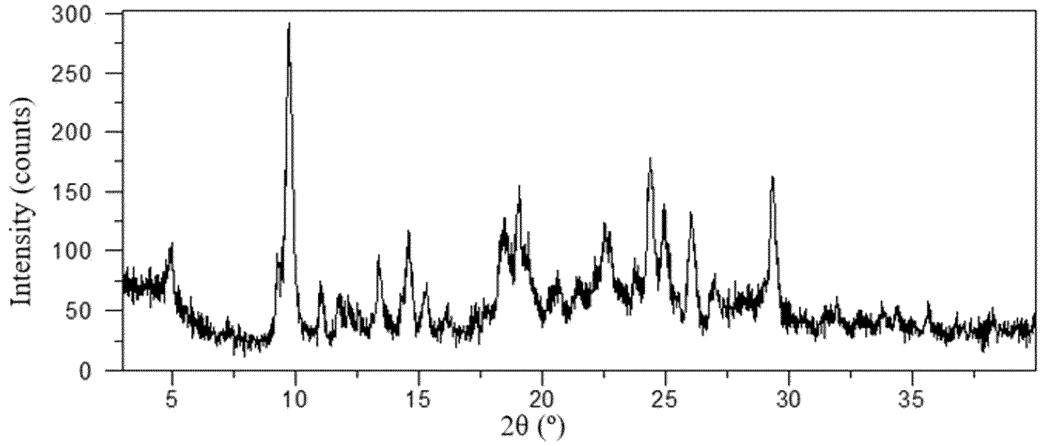


Figure.12

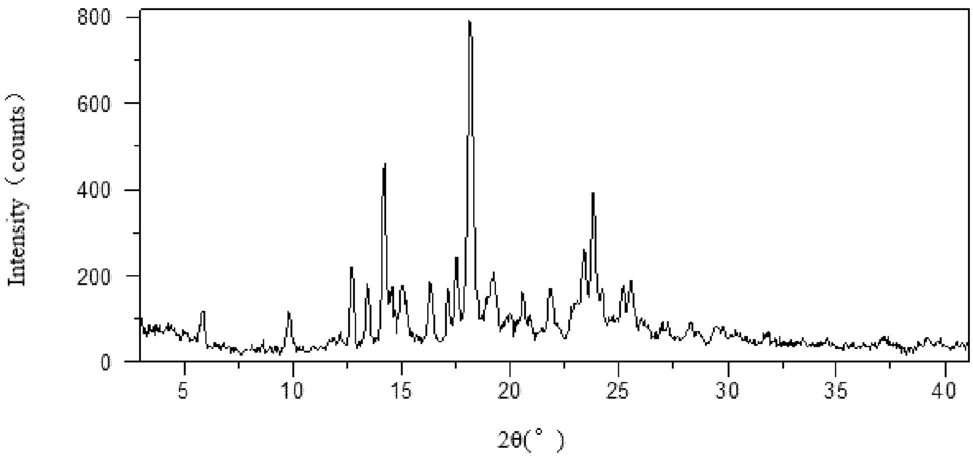


Figure.13

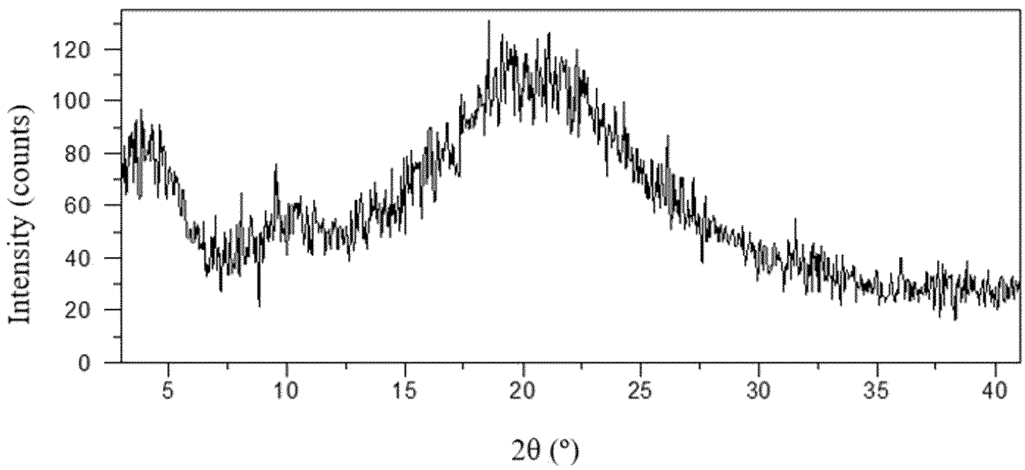


Figure.14

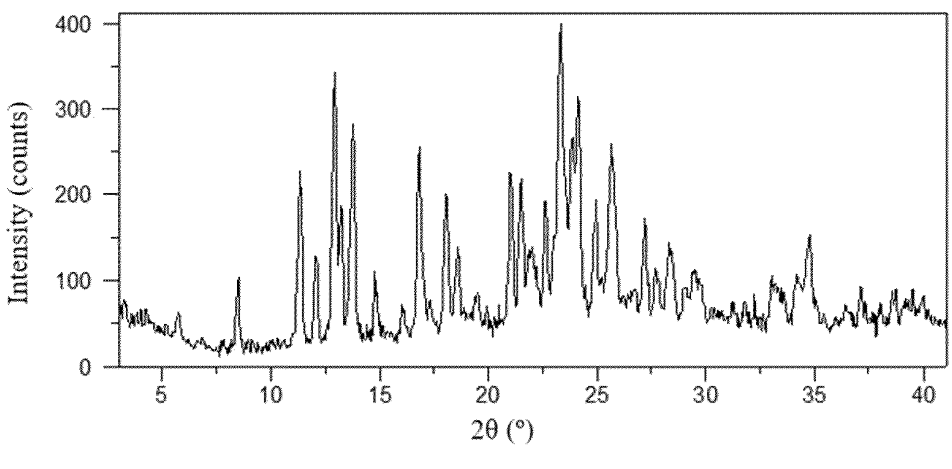


Figure.15

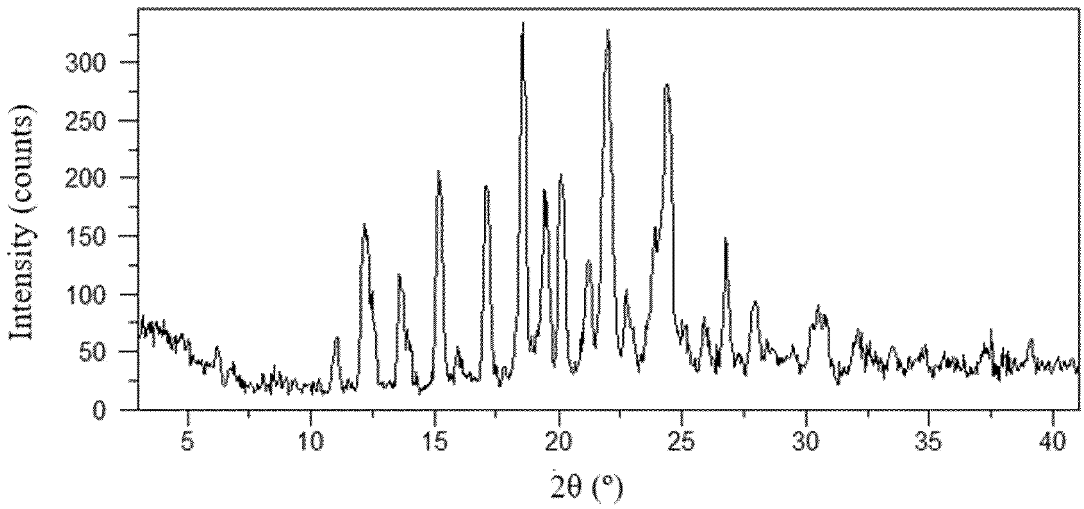


Figure.16

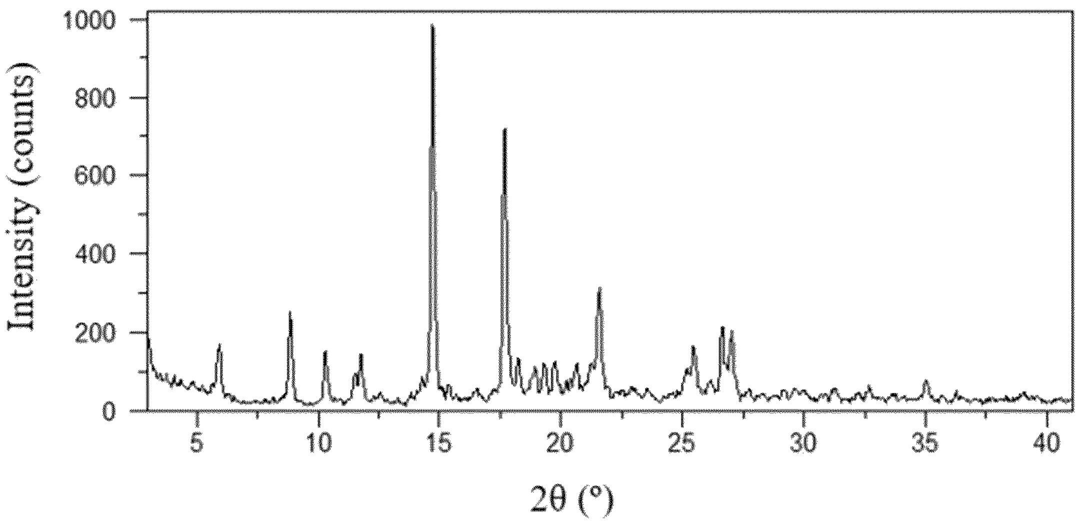


Figure.17

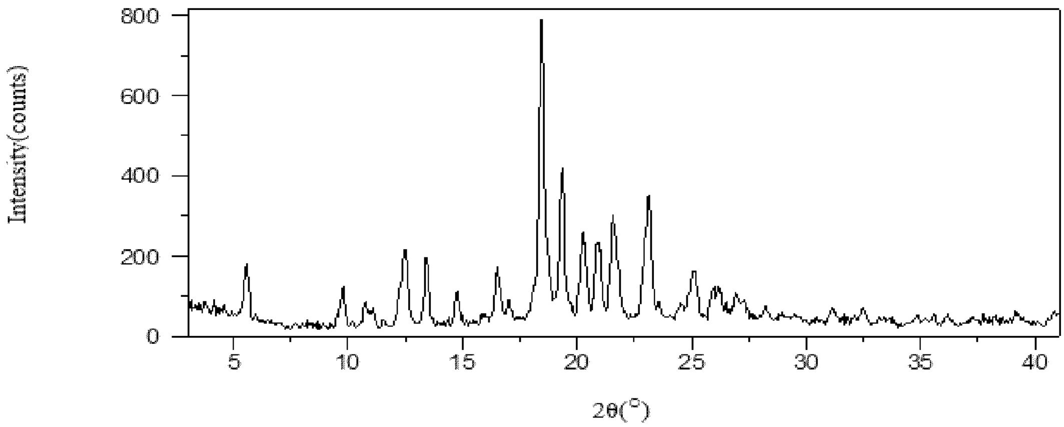


Figure.18

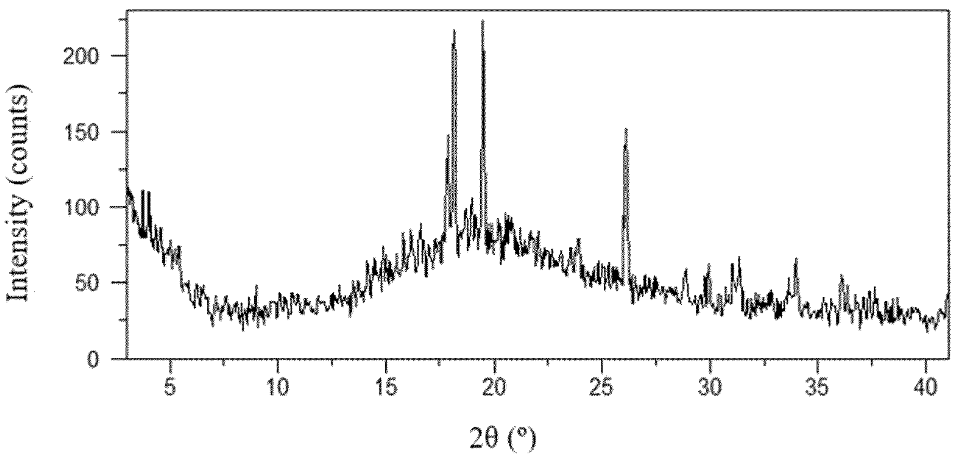


Figure.19

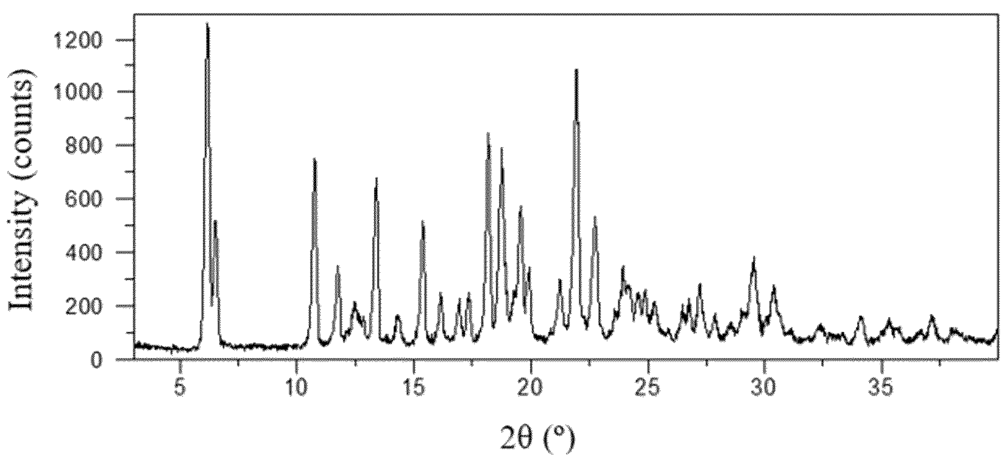


Figure.20

**INTERNATIONAL SEARCH REPORT**

International application No.
**PCT/CN2022/134410**

**A. CLASSIFICATION OF SUBJECT MATTER**

C07F9/6558(2006.01)i;A61K31/506(2006.01)i;A61P35/00(2006.01)i

According to International Patent Classification (IPC) or to both national classification and IPC

**B. FIELDS SEARCHED**

Minimum documentation searched (classification system followed by classification symbols)

C07F9/- A61K31/- A61P35/-

Documentation searched other than minimum documentation to the extent that such documents are included in the fields searched

Electronic data base consulted during the international search (name of data base and, where practicable, search terms used)

CNKI, CNTXT, DWPI, ENTXT, STN(CAPLUS, REGISTRY): 吡咯, 嘧啶, 苯基, 膦, 苯并, 轮烯, 受体酪氨酸激酶, ALX, RTK, UFO, ARK, Tyro7, 激酶, 抑制, 癌, 瘤, 对甲苯磺酸, 晶体, 晶型, 晶, +pyrrol+, +pyrimidin+, +benzo+, +benzocyclohepten+, kinase, inhibit+, toluenesulfonic acid, crystal+

**C. DOCUMENTS CONSIDERED TO BE RELEVANT**

| Category* | Citation of document, with indication, where appropriate, of the relevant passages | Relevant to claim No. |
|---|---|---|
| PX | WO 2021239133 A1 (NANJING CHIA TAI TIANQING PHARMACEUTICAL CO., LTD.) 02 December 2021 (2021-12-02)<br>entire document, in particular embodiment 115 | 1-11 |
| A | WO 2015038868 A1 (CEPHALON INC.) 19 March 2015 (2015-03-19)<br>entire document, in particular description, pages 38 and 46 | 1-11 |
| A | CN 106458914 A (CHANGZHOU JIEKAI PHARMATECH CO., LTD.) 22 February 2017 (2017-02-22)<br>entire document | 1-11 |
| A | CN 101796046 A (ASTRAZENECA AB) 04 August 2010 (2010-08-04)<br>entire document | 1-11 |
| A | CN 102356075 A (RIGEL PHARMACEUTICALS, INC.) 15 February 2012 (2012-02-15)<br>entire document | 1-11 |
| A | WO 2018102366 A1 (ARIAD PHARMACEUTICALS, INC.) 07 June 2018 (2018-06-07)<br>entire document | 1-11 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 February 2023** | **24 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/134410**

**C. DOCUMENTS CONSIDERED TO BE RELEVANT**

| Category* | Citation of document, with indication, where appropriate, of the relevant passages | Relevant to claim No. |
|---|---|---|
| A | WO 2020253860 A1 (JIANGSU HANSOH PHARMACEUTICAL GROUP CO., LTD. et al.) 24 December 2020 (2020-12-24) entire document | 1-11 |
| A | WO 2021125803 A1 (KOREA RESEARCH INSTITUTE OF CHEMICAL TECHNOLOGY) 24 June 2021 (2021-06-24) entire document | 1-11 |

Form PCT/ISA/210 (second sheet) (July 2022)

**INTERNATIONAL SEARCH REPORT**
**Information on patent family members**

International application No.
**PCT/CN2022/134410**

| Patent document cited in search report | | | Publication date (day/month/year) | Patent family member(s) | | | Publication date (day/month/year) |
|---|---|---|---|---|---|---|---|
| WO | 2021239133 | A1 | 02 December 2021 | CN | 115697993 | A | 03 February 2023 |
| WO | 2015038868 | A1 | 19 March 2015 | | None | | |
| CN | 106458914 | A | 22 February 2017 | WO | 2015143692 | A1 | 01 October 2015 |
| | | | | US | 2017137426 | A1 | 18 May 2017 |
| CN | 101796046 | A | 04 August 2010 | NI | 201000011 | A | 07 December 2010 |
| | | | | MX | 2010000658 | A | 26 March 2010 |
| | | | | CO | 6260075 | A2 | 22 March 2011 |
| | | | | KR | 20100042272 | A | 23 April 2010 |
| | | | | EP | 2183242 | A2 | 12 May 2010 |
| | | | | DOP | 2010000022 | A | 15 February 2010 |
| | | | | ZA | 201000107 | B | 29 September 2010 |
| | | | | JP | 2010533700 | A | 28 October 2010 |
| | | | | WO | 2009010789 | A2 | 22 January 2009 |
| | | | | WO | 2009010789 | A3 | 07 May 2009 |
| | | | | EA | 201000101 | A1 | 30 August 2010 |
| | | | | CR | 11220 | A | 20 May 2010 |
| | | | | CA | 2693880 | A1 | 22 January 2009 |
| | | | | AU | 2008277446 | A1 | 22 January 2009 |
| | | | | ECSP | 109958 | A | 31 March 2010 |
| | | | | US | 2009023719 | A1 | 22 January 2009 |
| | | | | US | 7718653 | B2 | 18 May 2010 |
| | | | | BRPI | 0814821 | A2 | 03 February 2015 |
| | | | | SV | 2010003459 | A | 30 April 2010 |
| CN | 102356075 | A | 15 February 2012 | PT | 2389372 | E | 07 January 2016 |
| | | | | SG | 172885 | A1 | 29 August 2011 |
| | | | | WO | 2010085684 | A1 | 29 July 2010 |
| | | | | ES | 2473584 | T3 | 07 July 2014 |
| | | | | SMT | 201500308 | B | 08 January 2016 |
| | | | | US | 2013310364 | A1 | 21 November 2013 |
| | | | | US | 9084795 | B2 | 21 July 2015 |
| | | | | US | 2014221352 | A1 | 07 August 2014 |
| | | | | US | 9248132 | B2 | 02 February 2016 |
| | | | | EP | 2565193 | A1 | 06 March 2013 |
| | | | | EP | 2565193 | B1 | 19 March 2014 |
| | | | | SI | 2389372 | T1 | 29 February 2016 |
| | | | | HUE | 026315 | T2 | 28 June 2016 |
| | | | | US | 2017137411 | A1 | 18 May 2017 |
| | | | | US | 10005767 | B2 | 26 June 2018 |
| | | | | US | 2018265503 | A1 | 20 September 2018 |
| | | | | US | 10556891 | B2 | 11 February 2020 |
| | | | | JP | 2012515793 | A | 12 July 2012 |
| | | | | JP | 5802136 | B2 | 28 October 2015 |
| | | | | HRP | 20151325 | T1 | 01 January 2016 |
| | | | | ES | 2555491 | T3 | 04 January 2016 |
| | | | | AU | 2010206683 | A1 | 11 August 2011 |
| | | | | AU | 2010206683 | B2 | 05 May 2016 |
| | | | | SG | 2014005318 | A | 28 March 2014 |
| | | | | HK | 1183023 | A1 | 13 December 2013 |
| | | | | HK | 1163076 | A1 | 07 September 2012 |
| | | | | EA | 201190082 | A1 | 28 February 2012 |

Form PCT/ISA/210 (patent family annex) (July 2022)

**INTERNATIONAL SEARCH REPORT**
**Information on patent family members**

International application No.
**PCT/CN2022/134410**

| Patent document cited in search report | Publication date (day/month/year) | Patent family member(s) | Publication date (day/month/year) |
|---|---|---|---|
| | | EA 022802 B1 | 31 March 2016 |
| | | US 2014011770 A1 | 09 January 2014 |
| | | US 8735400 B2 | 27 May 2014 |
| | | US 2012010406 A1 | 12 January 2012 |
| | | US 8563716 B2 | 22 October 2013 |
| | | PE 20110924 A1 | 24 December 2011 |
| | | NZ 594323 A | 31 May 2013 |
| | | MX 2011007750 A | 29 September 2011 |
| | | US 2012046245 A1 | 23 February 2012 |
| | | US 8563569 B2 | 22 October 2013 |
| | | PL 2389372 T3 | 29 February 2016 |
| | | DK 2389372 T3 | 14 December 2015 |
| | | SI 2565193 T1 | 31 July 2014 |
| | | HRP 20140454 T1 | 20 June 2014 |
| | | EP 2389372 A1 | 30 November 2011 |
| | | EP 2389372 B1 | 09 September 2015 |
| | | BRPI 1006942 A2 | 12 April 2016 |
| | | KR 20110111496 A | 11 October 2011 |
| | | KR 101762967 B1 | 28 July 2017 |
| | | US 2015307515 A1 | 29 October 2015 |
| | | US 9566283 B2 | 14 February 2017 |
| | | IL 214208 A0 | 31 August 2011 |
| | | IL 214208 A | 31 August 2015 |
| | | US 2012016122 A1 | 19 January 2012 |
| | | US 8530656 B2 | 10 September 2013 |
| | | CA 2749217 A1 | 29 July 2010 |
| | | CA 2749217 C | 21 March 2017 |
| | | PT 2565193 E | 05 June 2014 |
| | | US 2010190770 A1 | 29 July 2010 |
| | | US 8324200 B2 | 04 December 2012 |
| | | US 2012016131 A1 | 19 January 2012 |
| | | US 8507675 B2 | 13 August 2013 |
| | | US 2012010407 A1 | 12 January 2012 |
| | | US 8524901 B2 | 03 September 2013 |
| | | PL 2565193 T3 | 31 July 2014 |
| | | DK 2565193 T3 | 16 June 2014 |
| | | US 2020216431 A1 | 09 July 2020 |
| | | PE 20150621 A1 | 07 May 2015 |
| | | SMT 201400071 B | 07 July 2014 |
| WO 2018102366 A1 | 07 June 2018 | MA 46942 A | 05 May 2021 |
| | | US 2019382379 A1 | 19 December 2019 |
| | | US 11180482 B2 | 23 November 2021 |
| | | JP 2020503271 A | 30 January 2020 |
| | | JP 7025426 B2 | 24 February 2022 |
| | | EP 3548479 A1 | 09 October 2019 |
| WO 2020253860 A1 | 24 December 2020 | WO 2020253862 A1 | 24 December 2020 |
| | | TW 202115042 A | 16 April 2021 |
| WO 2021125803 A1 | 24 June 2021 | AU 2020406824 A1 | 11 August 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ROBINSON, DR et al.** *Oncogene,* 2000, vol. 19, 5548-5557
- **SEGALINY, AI et al.** *J. Bone Oncol,* 2015, vol. 4, 1-12
- **LINGER, R. M et al.** *Ther. Targets,* 2010, vol. 14 (10), 1073-1090
- **RESCIGNO, J. et al.** *Oncogene,* 1991, vol. 6 (10), 1909-1913
- **WU YM ; ROBINSON DR ; KUNG HJ.** *Cancer Res,* 2004, vol. 64 (20), 7311-7320
- **HUNG BI et al.** *DNA Cell Biol,* 2003, vol. 22 (8), 533-540
- Chinese Pharmacopoeia. 2020